# EUROPEAN PATENT SPECIFICATION

(11) **EP 2 547 288 B1**
(45) Date of publication and mention of the grant of the patent: **03.07.2019**
(21) Application number: 11756951.7
(22) Date of filing: 16.03.2011
(51) Int. Cl.: A61F 2/06, A61L 27/38, A61L 27/40, C12N 5/071

(54) **MULTILAYERED VASCULAR TUBES**
MEHRSCHICHTIGE GEFÄSSRÖHREN
TUBES VASCULAIRES MULTICOUCHES

(30) Priority: 16.03.2010 US 314238 P; 26.05.2010 GB 201008781
(43) Date of publication of application: 23.01.2013
(73) Proprietor: Organovo, Inc., San Diego, CA 92121 (US)
(72) Inventor: KHATIWALA, Chirag, San Diego CA 92126 (US); MURPHY, Keith, Los Angeles CA 90049 (US); SHEPHERD, Benjamin, San Diego CA 92102 (US)
(74) Representative: Avidity IP
(86) International application number: PCT/US2011/028713
(87) International publication number: WO 2011/116125

(56) References cited:
- US-A1- 2005 009 178
- US-A1- 2007 299 508
- US-A1- 2008 097 575
- US-A1- 2009 076 531
- ITO AKIRA ET AL: "NOVEL METHODOLOGY FOR FABRICATION OF TISSUE-ENGINEERED TUBULAR CONSTRUCTS USING MAGNETITE NANOPARTICLES AND MAGNETIC FORCE", TISSUE ENGINEERING, LARCHMONT, NY, US, vol. 11, no. 9-10, September 2005 (2005-09), pages 1553-1561, XP009081986, ISSN: 1076-3279, DOI: 10.1089/TEN.2005.11.1553
- NOROTTE C ET AL: "Scaffold-free vascular tissue engineering using bioprinting", BIOMATERIALS, ELSEVIER SCIENCE PUBLISHERS BV., BARKING, GB, vol. 30, no. 30, October 2009 (2009-10), pages 5910-5917, XP026524655, ISSN: 0142-9612, DOI: 10.1016/J.BIOMATERIALS.2009.06.034 [retrieved on 2009-08-06]
- Somali Chaterji ET AL: "Scaffold-Free In Vitro Arterial Mimetics: The Importance of Smooth Muscle-Endothelium Contact", Tissue Engineering Part A, 8 March 2010 (2010-03-08), pages 1901-1912, XP055097101, New Rochelle DOI: 10.1089/ten.tea.2009.0271 Retrieved from the Internet: URL:http://search.proquest.com/docview/504 750554 [retrieved on 2014-01-17]
- KELM JENS M ET AL: "Design of custom-shaped vascularized tissues using microtissue spheroids as minimal building units", TISSUE ENGINEERING, LARCHMONT, NY, US, vol. 12, no. 8, August 2006 (2006-08), pages 2151-2160, XP002545431, ISSN: 1076-3279
- L'HEUREUX N ET AL: "A completely biological tissue-engineered human blood vessel", FASEB JOURNAL, FED. OF AMERICAN SOC. FOR EXPERIMENTAL BIOLOGY, US, vol. 12, no. 1, January 1998 (1998-01), pages 47-56, XP008132869, ISSN: 0892-6638

## Description

### BACKGROUND OF THE INVENTION

In the United States, there is a need for replacement blood vessels for diseased arteries or replacement abdominal organs, particularly for small diameter vascular grafts. Small arteries with diameters less than six millimeters cannot be replaced with artificial materials due to high rates of thrombosis (Connolly et al. (1988); Greisler et al. (1988)).

C. Norrotte et al. ["Scaffold-free vacular tissue engineering using bioprinting", Biomaterials 30 (2009), 5910-5917], disclose rapid prototyping bioprinting methods for scaffold-free small diameter vascular reconstruction.

### SUMMARY OF THE INVENTION

The scope of the invention is defined by the claims. Any references in the description to methods of treatment refer to the compounds, compositions, constructs of the present invention for use in a method for treatment of the human (or animal) body by therapy.

Described herein are engineered multilayer vascular tubes, methods for producing such tubes, and therapeutic, diagnostic, and research uses for such tubes. The engineered vascular tubes described herein are free of any pre-formed scaffold because pre-formed scaffolds are not utilized in the production of the engineered multilayer vascular tubes described herein. This innovation provides, among other things, viable engineered multilayer vascular tubes that are free of pre-formed scaffold materials, and are accordingly therapeutically acceptable alternatives to non-engineered multilayer vascular tubes.

According to the invention, described herein are engineered multilayered vascular tubes formed through the use of a bioprinter comprising at least one layer of differentiated adult fibroblasts, at least one layer of differentiated adult smooth muscle cells, wherein any layer further comprises differentiated adult endothelial cells, wherein said tube has a ratio of endothelial cells to smooth muscle cells of 5:95 to 45:55; and wherein said engineered multilayered vascular tube has one, two, three or all of the following features: (a) the engineered multilayered vascular tube is compliant; (b) the internal diameter of the engineered multilayered vascular tube is 6 mm or smaller; (c) the length of the tube is up to 30 cm; and (d) the thickness of the engineered multilayered vascular tube is substantially uniform along a region of the tube; provided that the engineered multilayered vascular tube is free of any pre-formed scaffold.

In one embodiment, the fibroblasts, smooth muscle cells, and endothelial cells are autologous. In another embodiment, the burst strength is sufficient to withstand physiological blood pressure. In yet another embodiment, the lumen of the tube was formed from a removable lumen-forming filler body. In yet another embodiment, the ratio of endothelial cells to smooth muscle cells is 5:95 to about 25:75. In yet another embodiment, the ratio of endothelial cells to smooth muscle cells is about 15:85. In yet another embodiment, the engineered multilayered vascular tube is compliant. In yet another embodiment, the internal diameter of the engineered multilayer vascular tube is 6 mm or smaller. In yet another embodiment, the internal diameter of the engineered multilayered vascular tube is about 0.5 mm or smaller. In yet another embodiment, the thickness of the engineered multilayered vascular tube is substantially uniform over a region of the tube. In yet another embodiment, the thickness of the engineered multilayered vascular tube is substantially uniform over the length of the tube. In yet another embodiment, the tube is substantially free of non-differentiated adult fibroblasts. In yet another embodiment, the tube is substantially free of non-differentiated adult smooth muscle cells. In yet another embodiment, the tube is substantially free of non-differentiated adult endothelial cells. In yet another embodiment, the tube has a branched structure. In yet another embodiment, the tube further comprises magnetic particles. In yet another embodiment, the tube is for use in bypass grafting. In yet another embodiment, the tube is for use in arteriovenous access. In yet another embodiment, the tube is for use in drug testing. In yet another embodiment, the tube is for use in cardiovascular device testing. In yet another embodiment, the cardiovascular device is a stent. In some embodiments, the engineered multilayered vascular tube is non-innervated. In some embodiments, said differentiated adult fibroblasts are non-vascular fibroblasts.

According to the invention are methods of forming an engineered multilayered vascular tube, comprising: positioning at least one body of filler matrix, at least one multicellular body of smooth muscle cells wherein the cells are cohered to one another, at least one multicellular body of endothelial cells wherein the cells are cohered to one another, and at least one multicellular body of fibroblast cells wherein the cells are cohered to one another to form a desired vascular tube structure, wherein the ratio of endothelial cells to smooth muscle cells in the engineered multilayered vascular tube is 5:95 to 45:55, and wherein one or more filler matrix bodies form the lumen of the tube and a plurality of filler matrix bodies surround the tube; and allowing the multicellular bodies of the engineered multilayer vascular tube to cohere; provided that no pre-formed scaffold is used at any time, and provided that the engineered multilayered tube is formed through use of a bioprinter.

According to the invention the ratio of endothelial cells to smooth muscle cells in the engineered multilayer vascular tube is 5:95 to 45:55. In another embodiment, the ratio of endothelial cells to smooth muscle cells in the engineered multilayer vascular tube is 5:95 to about 25:75. In yet another embodiment, the ratio of endothelial cells to smooth muscle cells in the engineered multilayer vascular tube is about 15:85. In yet another embodiment, the lumen of the tube is 6 mm or smaller. In yet another embodiment, the lumen of the tube is about 0.5 mm or smaller. In yet another embodiment, the smooth muscle cells, endothelial cells, and/or the fibroblasts are autologous. In yet another embodiment, the smooth muscle cells, endothelial cells, and/or the fibroblasts are human adult differentiated cells. In yet another embodiment, the method further comprises culturing the engineered multilayer vascular tube in a maturation chamber. In some embodiments, one or more of the multicellular bodies of cells are elongate. In some embodiments, one or more of the multicellular bodies of cells are substantially spherical.

In another aspect are engineered multilayered vascular tubes formed through the use of a bioprinter comprising at least one layer of differentiated adult fibroblasts, at least one layer of differentiated adult smooth muscle cells, wherein any layer further comprises differentiated adult endothelial cells, wherein said tube has the following features: (a) a ratio of endothelial cells to smooth muscle cells of 5:95 to about 25:75; (b) the engineered multilayered vascular tube is compliant; (c) the internal diameter of the engineered multilayered vascular tube is 6 mm or smaller; (d) the length of the tube is up to 30 cm; and (e) the thickness of the engineered multilayered vascular tube is substantially uniform along a region of the tube; provided that the engineered multilayered vascular tube is free of any pre-formed scaffold.

In one embodiment, the burst strength is sufficient to withstand physiological blood pressure. In another embodiment, the lumen of the tube was formed from a lumen-forming filler body. In yet another embodiment, the ratio of endothelial cells to smooth muscle cells is about 15:85. In yet another embodiment, the thickness of the engineered multilayered vascular tube is substantially uniform over the entire length of the tube. In yet another embodiment, the tube further comprises magnetic particles. In yet another embodiment, the tube has a branched structure. In yet another embodiment, the tube is for use in bypass grafting. In yet another embodiment, the tube is for use in arteriovenous access. In yet another embodiment, the tube is for use in drug testing. In yet another embodiment, the tube is for use in cardiovascular device testing. In yet another embodiment, the cardiovascular device is a stent. In some embodiments, the engineered multilayered vascular tube is non-innervated. In some embodiments, said differentiated adult fibroblasts are non-vascular fibroblasts.

In another aspect are engineered multilayered vascular tubes formed through the use of a bioprinter comprising an outer layer of differentiated adult fibroblasts, at least one inner layer of differentiated adult smooth muscle cells and differentiated adult endothelial cells, and wherein said tube has the following features: (a) a ratio of endothelial cells to smooth muscle cells of 5:95 to 45:55; (b) the internal diameter of the engineered multilayered vascular tube is 6 mm or smaller; (c) the length of the tube is up to 30 cm; and (d) the thickness of the engineered multilayered vascular tube is substantially uniform along a region of the tube; provided that the engineered multilayered vascular tube is free of any pre-formed scaffold.

In one embodiment, the ratio of endothelial cells to smooth muscle cells is 5:95 to about 25:75. In another embodiment, the ratio of endothelial cells to smooth muscle cells is about 15:85. In yet another embodiment, the internal diameter of the engineered multilayer vascular tube is 6 mm or smaller. In yet another embodiment, the internal diameter of the engineered multilayered vascular tube is about 0.5 mm or smaller. In yet another embodiment, the engineered multilayered vascular tube is compliant. In yet another embodiment, the tube has a branched structure. In yet another embodiment, the length of the tube is about 1 cm to 30 cm. In yet another embodiment, the thickness of the tube is substantially uniform along a region of the tube. In yet another embodiment, the thickness of the tube is substantially uniform along the length of the tube. In yet another embodiment, the tube has an outer support structure. In yet another embodiment, the tube is substantially free of non-differentiated adult fibroblasts. In yet another embodiment, the tube is substantially free of non-differentiated adult smooth muscle cells. In yet another embodiment, the tube is substantially free of non-differentiated adult endothelial cells. In yet another embodiment, the tube comprises magnetic particles. In some embodiments, the engineered multilayered vascular tube is non-innervated. In some embodiments, said differentiated adult fibroblasts are non-vascular fibroblasts.

In another aspect are methods for treating a patient, comprising providing an engineered multilayered vascular tube formed through the use of a bioprinter comprising at least one layer of differentiated adult fibroblasts, at least one layer of differentiated adult smooth muscle cells, wherein any layer further comprises differentiated adult endothelial cells, wherein said tube has the following features: (a) a ratio of endothelial cells to smooth muscle cells of 5:95 to 45:55; (b) the engineered multilayered vascular tube is compliant; (c) the internal diameter of the engineered multilayered vascular tube is 6 mm or smaller; (d) the length of the tube is up to 30 cm; and (e) the thickness of the engineered multilayered vascular tube is substantially uniform along a region of the tube; provided that the engineered multilayered vascular tube is free of any pre-formed scaffold.

In one embodiment, the fibroblasts, smooth muscle cells, and endothelial cells of the engineered multilayered vascular tube are autologous. In another embodiment, the ratio of endothelial cells to smooth muscle cells of the engineered multilayered vascular tube is about 15:85. In yet another embodiment, the burst strength of the engineered multilayered vascular tube is sufficient to withstand physiological blood pressure. According to the invention the lumen of the engineered multilayered vascular tube was formed from a lumen-forming filler body. In yet another embodiment, the engineered multilayered vascular tube is compliant. In yet another embodiment, the internal diameter of the engineered multilayer vascular tube is 6 mm or smaller. In yet another embodiment, the internal diameter of the engineered multilayered vascular tube is about 0.5 mm or smaller. In yet another embodiment, the length of the tube is from about 1 cm to 30 cm. In yet another embodiment, the thickness of the engineered multilayered vascular tube is substantially uniform along a region of the tube. In yet another embodiment, the thickness of the engineered multilayered vascular tube is substantially uniform along the length of the tube. In yet another embodiment, the engineered multilayered vascular tube has a branched structure. In yet another embodiment, the engineered multilayered vascular tube further comprises magnetic particles. In some embodiments, the engineered multilayered vascular tube is non-innervated. In some embodiments, said differentiated adult fibroblasts are non-vascular fibroblasts. In yet another embodiment, the patient is in need of a coronary or peripheral bypass. In yet another embodiment, the patient is in need of hemodialysis. In yet another embodiment, the patient in need has end-stage renal disease. In yet another embodiment, the patient in need has diabetes. In yet another embodiment, the patient in need has arteriosclerosis. In yet another embodiment, the patient in need has congenital heart birth defects.

In another aspect are engineered multilayered vascular tubes formed through the use of a bioprinter comprising an outer layer of differentiated adult fibroblasts, at least one inner layer of differentiated adult smooth muscle cells and differentiated adult endothelial cells, and having the following features: (a) a ratio of endothelial cells to smooth muscle cells of 5:95 to about 25:75; (b) the engineered multilayered vascular tube is compliant; (c) the internal diameter of the engineered multilayered vascular tube is 6 mm or smaller; (d) the length of the tube is about 1 cm to 30 cm; and (e) the thickness of the engineered multilayered vascular tube is substantially uniform along a region of the tube; provided that the engineered multilayered vascular tube is free of any pre-formed scaffold.

In one embodiment, the burst strength is sufficient to withstand physiological blood pressure. In another embodiment, the tube was formed from a lumen-forming filler body. In another embodiment, the ratio of endothelial cells to smooth muscle cells is about 15:85. In another embodiment, the thickness of the engineered multilayered vascular tube is substantially uniform over the entire length of the tube. In another embodiment, the tube is substantially free of non-differentiated adult fibroblasts. In another embodiment, the tube is substantially free of non-differentiated adult smooth muscle cells. In another embodiment, the tube is substantially free of non-differentiated adult endothelial cells. In another embodiment, the tube comprises magnetic particles. In another embodiment, the tube has a branched structure. In some embodiments, the engineered multilayered vascular tube is non-innervated. In some embodiments, said differentiated adult fibroblasts are non-vascular fibroblasts. In another embodiment, the tube is for use in bypass grafting. In another embodiment, the tube is for use in arteriovenous access. In another embodiment, the tube is for use in drug testing. In another embodiment, the tube is for use in cardiovascular device testing. In another embodiment, the cardiovascular device is a stent. In one embodiment, the ratio of endothelial cells to smooth muscle cells in the engineered multilayer vascular tube is 5:95 to about 25:75. In another embodiment, the ratio of endothelial cells to smooth muscle cells in the engineered multilayer vascular tube is about 15:85. In another embodiment, the lumen of the tube is 6 mm or smaller. In another embodiment, the smooth muscle cells, endothelial cells, and/or the fibroblasts are human adult differentiated cells. In another embodiment, the method comprises culturing the engineered multilayer vascular tube in a maturation chamber.

In another aspect are engineered multilayered vascular tubes formed through the use of a bioprinter comprising an outer layer of differentiated adult fibroblasts, at least one inner layer of differentiated adult smooth muscle cells and differentiated adult endothelial cells, and having the following features: (a) a removable lumen-forming filler body; (b) the ratio of endothelial cells to smooth muscle cells is 5:95 to about 25:75; (c) the internal diameter of the engineered multilayered vascular tube is 6 mm or smaller; (d) the length of the tube is about 1 cm to 30 cm; (e) the thickness of the engineered multilayered vascular tube is substantially uniform; and (f) an outer support structure; provided that the engineered multilayered vascular tube is non-innervated and free of any pre-formed scaffold.

In one embodiment, the outer support structure is removed. In another embodiment, the lumen-forming filler body is removed. In another embodiment, the ratio of endothelial cells to smooth muscle cells is about 15:85. In another embodiment, the engineered multilayered vascular tube is compliant. In another embodiment, the tube has a branched structure.

In another aspect are engineered vascular tubes formed through use of a bioprinter comprising differentiated adult smooth muscle cells, differentiated adult endothelial cells, and differentiated adult fibroblasts, wherein said cells are cohered to one another, and wherein said tube has a ratio of endothelial cells to smooth muscle cells of 5:95 to 45:55; and wherein said tube has one, two, or all of the following features: (a) the internal diameter of the engineered vascular tube is 6 mm or smaller; (b) the length of the tube is up to 30 cm; and (c) the thickness of the engineered vascular tube is substantially uniform along a region of the tube; provided that the engineered vascular tube is free of any pre-formed scaffold.

In some embodiments, the differentiated adult smooth muscle cells, differentiated adult endothelial cells, and differentiated adult fibroblasts are uniformly distributed in the engineered vascular tube. In other embodiments, the differentiated adult smooth muscle cells, differentiated adult endothelial cells, and differentiated adult fibroblasts are non-uniformly distributed in the engineered vascular tube. In further embodiments, the cells are distributed in layers. In still further embodiments, one or more layers are characterized by a distribution of cell types that is different from one or more other layers. In some embodiments, the cells are non-uniformly distributed at the time the engineered vascular tube is formed. In some embodiments, one or more cell types migrate or segregate to some degree thus creating a non-uniform distribution.

In some embodiments, the engineered vascular tube is compliant. In one embodiment, the fibroblasts, smooth muscle cells, and endothelial cells are autologous. In another embodiment, the burst strength is sufficient to withstand physiological blood pressure. In yet another embodiment, the lumen of the tube was formed from a removable lumen-forming filler body. In yet another embodiment, the ratio of endothelial cells to smooth muscle cells is 5:95 to about 25:75. In yet another embodiment, the ratio of endothelial cells to smooth muscle cells is about 15:85. In yet another embodiment, the engineered multilayered vascular tube is compliant. In yet another embodiment, the internal diameter of the engineered multilayer vascular tube is 6 mm or smaller. In yet another embodiment, the internal diameter of the engineered multilayered vascular tube is about 0.5 mm or smaller. In yet another embodiment, the thickness of the engineered multilayered vascular tube is substantially uniform over a region of the tube. In yet another embodiment, the thickness of the engineered multilayered vascular tube is substantially uniform over the length of the tube. In yet another embodiment, the tube is substantially free of non-differentiated adult fibroblasts. In yet another embodiment, the tube is substantially free of non-differentiated adult smooth muscle cells. In yet another embodiment, the tube is substantially free of non-differentiated adult endothelial cells. In yet another embodiment, the tube has a branched structure. In yet another embodiment, the tube further comprises magnetic particles. In yet another embodiment, the tube is for use in bypass grafting. In yet another embodiment, the tube is for use in arteriovenous access. In yet another embodiment, the tube is for use in drug testing. In yet another embodiment, the tube is for use in cardiovascular device testing. In yet another embodiment, the cardiovascular device is a stent. In some embodiments, the engineered multilayered vascular tube is non-innervated. In some embodiments, said differentiated adult fibroblasts are non-vascular fibroblasts.

The terms "cohere," "cohered," and "cohesion" as used herein, refer to cell-cell adhesion properties that bind cells, cell aggregates, multicellular bodies, and/or layers. The terms are used interchangeably with "fuse," "fused," and "fusion."

The term "scaffold" as used herein, refers to synthetic scaffolds such as polymer scaffolds, non-synthetic scaffolds such as extracellular matrix layers, and any other type of pre-formed scaffold that is integral to the physical structure of the engineered multilayered vascular tube and not removed from the tube.

### BRIEF DESCRIPTION OF THE DRAWINGS

The novel features of the invention are set forth in the appended claims. A better understanding of the features and advantages of the present invention will be obtained by reference to the following detailed description that sets forth illustrative embodiments, in which the principles of the invention are utilized, and the accompanying drawings of which:
**FIG. 1** is an illustrative hematoxylin and eosin staining of a HASMC-HAEC (human aortic smooth muscle cells-human aortic endothelial cells) mixed cell cylinder.
**FIG. 2** is an illustrative geometrical arrangement for constructing a vascular tube having an outer layer of HDF and an inner layer of HASMC-HAEC using HDF multicellular bodies and HASMC-HAEC mixed multicellular bodies.
**FIG. 3A** is an illustrative un-isolated multilayered vascular tube.
**FIG. 3B** is an illustrative isolated multilayered vascular tube, formed by cohesion between cylinders encapsulated within agarose support structure at 24 hours post-incubation.

### DETAILED DESCRIPTION OF THE INVENTION

The invention relates to the field of regenerative medicine and tissue engineering. More particularly, the invention relates to the production of multilayered vascular tubes comprising fibroblasts, smooth muscle cells, and endothelial cells and having particular features.

Tissue engineering provides solutions to problems caused by growing demand for organ replacement coupled with shortage of transplantable organs, including blood vessels. (Langer and Vacanti (1993)). In the United States, there is a need for replacement blood vessels for diseased arteries or replacement abdominal organs, particularly for small diameter vascular grafts. Recognized also is a need for improved blood vessels for research uses. Small arteries with diameters less than five to six mm cannot be replaced with artificial materials due to high rates of thrombosis (Connolly et al. (1988); Greisler et al. (1988)). Tissue engineered vascular grafts provide a viable method of addressing the shortage of native transplantable blood vessels, and also in applications such as cardiovascular device testing and drug testing, for example.

Native vessels comprise three layers: an inner layer (intima) consisting of endothelial cells, a middle layer (media) of vascular smooth muscle, and an outer layer (adventitia) of fibroblasts. Ideal tissue engineered vascular grafts should be free of a synthetic scaffold that could otherwise affect long term behavior of the graft or interfere with its primary biological function. The multilayered vascular tubes described herein achieve this result. Small diameter tissue engineered vascular grafts are desired, having internal diameters at 6 mm or smaller. The multilayered vascular tubes described herein achieve this result. Others have not been able to create a synthetic scaffold-free compliant graft that can withstand physiological blood pressures. The multilayered vascular tubes described herein achieve this result. Others have not been able to recreate a vascular construct containing the three predominant cell types present in vascular tissues, fibroblasts, smooth muscle cells, and endothelial cells, without the presence of supporting biomaterials. The multilayered vascular tubes described herein achieve this result.

The assembly of a multilayered vascular construct tube comprising fibroblasts, smooth muscle cells, and endothelial cells that is compliant, substantially uniform, free of synthetic scaffolding, and having an internal diameter of about 0.5 mm or less has not yet been accomplished. The multilayered vascular tubes described herein achieve this result.

According to the invention, described herein are engineered multilayered vascular tubes formed through use of a bioprinter comprising at least one layer of differentiated adult fibroblasts, at least one layer of differentiated adult smooth muscle cells, wherein any layer further comprises differentiated adult endothelial cells, wherein said tube has a ratio of endothelial cells to smooth muscle cells of 5:95 to 45:55; and wherein said tube has at least one of the following features: (a) the engineered multilayered vascular tube is compliant; (b) the internal diameter of the engineered multilayered vascular tube is 6 mm or smaller; (c) the length of the tube is up to 30 cm; and (d) the thickness of the engineered multilayered vascular tube is substantially uniform along a region of the tube; provided that the engineered multilayered vascular tube is free of any pre-formed scaffold. In particular embodiments, the engineered multilayered vascular tube has at least two of these features. In yet more particular embodiments, the engineered multilayered vascular tube has at least three of these features. In even more particular embodiments, the engineered multilayered vascular tube has all of these features. In some embodiments, the engineered multilayered vascular tube is non-innervated. In some embodiments, said differentiated adult fibroblasts are non-vascular fibroblasts.

According to the invention, also described herein are methods of forming an engineered multilayered vascular tube, comprising: positioning at least one body of filler matrix, at least one multicellular body of smooth muscle cells wherein the cells are cohered to one another, at least one multicellular body of endothelial cells wherein the cells are cohered to one another, and at least one multicellular body of fibroblast cells wherein the cells are cohered to one another to form a desired vascular tube structure, wherein the ratio of endothelial cells to smooth muscle cells in the engineered multilayered tube is 5:95 to 45:55, and wherein one or more filler matrix bodies form the lumen of the tube and a plurality of filler matrix bodies surround the tube; and allowing the multicellular bodies of the engineered multilayer vascular tube to cohere; provided that no pre-formed scaffold is used at any time, and provided that the engineered multilayered vascular tube is formed through use of a bioprinter.

In one embodiment, the multicellular body is an elongate cellular body. The term elongate cellular body, as used throughout the text, is only provided as an example and not a limiting embodiment. Accordingly, those of skill in the art, using the disclosure herein, will be able to apply the teachings for an elongate cellular body to any other cellular body (e.g., non-elongate cellular bodies). In one embodiment, the cellular body is a substantially spherical cellular body.

Also described herein are engineered multilayered vascular tubes formed through the use of a bioprinter comprising at least one layer of differentiated adult fibroblasts, at least one layer of differentiated adult smooth muscle cells, wherein any layer further comprises differentiated adult endothelial cells, wherein said tube has the following features: (a) a ratio of endothelial cells to smooth muscle cells of 5:95 to about 25:75; (b) the engineered multilayered vascular tube is compliant; (c) the internal diameter of the engineered multilayered vascular tube is 6 mm or smaller; (d) the length of the tube is up to 30 cm; and (e) the thickness of the engineered multilayered vascular tube is substantially uniform along a region of the tube; provided that the engineered multilayered vascular tube is free of any pre-formed scaffold.

Also described herein are engineered multilayered vascular tubes formed through the use of a bioprinter comprising an outer layer of differentiated adult fibroblasts, at least one inner layer of differentiated adult smooth muscle cells and differentiated adult endothelial cells, and wherein said tube has a ratio of endothelial cells to smooth muscle cells of 5:95 to 45:55; and wherein said tube has at least one of the following features: (a) the internal diameter of the engineered multilayered vascular tube is 6 mm or smaller; (b) the length of the tube is up to 30 cm; and (c) the thickness of the engineered multilayered vascular tube is substantially uniform along a region of the tube; provided that the engineered multilayered vascular tube is free of any pre-formed scaffold. In particular embodiments, the engineered multilayered vascular tube has at least two of these features. In yet more particular embodiments, the engineered multilayered vascular tube has at least three of these features. In even more particular embodiments, the engineered multilayered vascular tube all of these features. In some embodiments, the engineered multilayered vascular tube is non-innervated. In some embodiments, said differentiated adult fibroblasts are non-vascular fibroblasts.

Further described herein are methods for treating a patient comprising providing an engineered multilayered vascular tube formed through the use of a bioprinter comprising at least one layer of differentiated adult fibroblasts, at least one layer of differentiated adult smooth muscle cells, wherein any layer further comprises differentiated adult endothelial cells, wherein said tube has the following features: (a) a ratio of endothelial cells to smooth muscle cells of 5:95 to 45:55; (b) the engineered multilayered vascular tube is compliant; (c) the internal diameter of the engineered multilayered vascular tube is 6 mm or smaller; (d) the length of the tube is up to 30 cm; and (e) the thickness of the engineered multilayered vascular tube is substantially uniform along a region of the tube; provided that the engineered multilayered vascular tube is free of any pre-formed scaffold.

Also described herein are engineered vascular tubes formed through the use of a bioprinter comprising differentiated adult smooth muscle cells, differentiated adult endothelial cells, and differentiated adult fibroblasts, wherein said cells are cohered to one another, and wherein said tube has the following features: (a) a ratio of endothelial cells to smooth muscle cells of 5:95 to 45:55; (b) the internal diameter of the engineered vascular tube is 6 mm or smaller; (c) the length of the tube is up to 30 cm; and (d) the thickness of the engineered vascular tube is substantially uniform along a region of the tube; provided that the engineered vascular tube is free of any pre-formed scaffold.

The cell types used in the engineered multilayered vascular tubes are optionally sourced from allogeneic tissues (cell or tissue that originates from or is derived from a donor of the same species as the recipient) or from autologous grafts (cell or tissue that originates with or is derived from the recipient), and are optionally fresh or cryopreserved. The cells may be differentiated from stem cells, e.g., a multipotent regenerative cell with the potential to differentiate into a variety of other cell types, which perform one or more specific functions and have the ability to self-renew, or progenitor cells, e.g., unipotent or multipotent regenerative cell with the potential to differentiate into at least one cell type and has limited or no ability to self-renew. The cells may also be sourced from, for example, human aortic cells, human umbilical cord cells, human adipose tissue, human bone marrow, human placenta or any other source from which human differentiated cells may be obtained. Preferably, the cell types used in the engineered multilayered vascular tubes described herein are adult differentiated cells.

The cell types used in the engineered multilayered vascular tubes may be cultured in any manner known in the art. Methods of cell and tissue culturing are known in the art, and are described, for example, in *Cell & Tissue Culture: Laboratory Procedures;* Freshney (1987), Culture of Animal Cells: A Manual of Basic Techniques. General mammalian cell culture techniques, cell lines, and cell culture systems that may be used in conjunction with the present invention are also described in Doyle, A., Griffiths, J. B., Newell, D. G., (eds.) Cell and Tissue Culture: Laboratory Procedures, Wiley (1998).

Appropriate growth conditions for mammalian cells in culture are well known in the art. Cell culture media generally include essential nutrients and, optionally, additional elements such as growth factors, salts, minerals, vitamins, etc., that may be selected according to the cell type(s) being cultured. Particular ingredients may be selected to enhance cell growth, differentiation, secretion of specific proteins, etc. In general, standard growth media include Dulbecco's Modified Eagle Medium, low glucose (DMEM), with 110 mg/L pyruvate and glutamine, supplemented with 10-20% fetal bovine serum (FBS) or calf serum and 100 U/ml penicillin, 0.1 mg/ml streptomycin are appropriate as are various other standard media well known to those in the art. Preferably cells are cultured under sterile conditions in an atmosphere of 3-15% CO₂, preferably 5% CO₂, at a temperature at or near the body temperature of the animal of origin of the cell. For example, human cells are preferably cultured at approximately 37°C.

The cells can also be cultured with cellular differentiation agents to induce differentiation of the cell along the desired line. For instance, cells can be cultured with growth factors, cytokines, etc. The term "growth factor" as used herein refers to a protein, a polypeptide, or a complex of polypeptides, including cytokines, that are produced by a cell and which can affect itself and/or a variety of other neighboring or distant cells. Typically growth factors affect the growth and/or differentiation of specific types of cells, either developmentally or in response to a multitude of physiological or environmental stimuli. Some, but not all, growth factors are hormones. Exemplary growth factors are insulin, insulin-like growth factor (IGF), nerve growth factor (NGF), vascular endothelial growth factor (VEGF), keratinocyte growth factor (KGF), fibroblast growth factors (FGFs), including basic FGF (bFGF), platelet-derived growth factors (PDGFs), including PDGF-AA and PDGF-AB, hepatocyte growth factor (HGF), transforming growth factor alpha (TGF-α), transforming growth factor beta (TGF-β), including TGFβ1 and TGFβ3, epidermal growth factor (EGF), granulocyte-macrophage colony-stimulating factor (GM-CSF), granulocyte colony-stimulating factor (G-CSF), interleukin-6 (IL-6), IL-8, and the like. Growth factors are discussed in, among other places, Molecular Cell Biology, Scientific American Books, Darnell et al., eds., 1986; Principles of Tissue Engineering, 2d ed., Lanza et al., eds., Academic Press, 2000. The skilled artisan will understand that any and all culture-derived growth factors in the conditioned media described herein are within the scope of the invention.

The fibroblasts, smooth muscle cells, and endothelial cells of the present invention may be cultured separately before being combined into a multicellular body for formation into the vascular tube. For example, the fibroblasts, smooth muscle cells, and endothelial cells can be separately grown in culture in tissue culture flasks, and when confluent, can be passaged and mixed together into one culture to form a mixed cell suspension, and centrifuged with supernatant removed to form a highly dense and compact cell pellet for a mixed cell paste. Suitable mixed cell suspensions include combinations of fibroblasts and smooth muscle cells, fibroblasts and endothelial cells, and smooth muscle cells and endothelial cells. The cells in the mixed cell suspension can be allowed to aggregate with one another and initiate cell-cell adhesions, for example, by incubation on an orbital shaker. The mixed cell paste can be formed into a multicellular body, for example, by aspiration into a capillary tube. Cellular cylinders can then be extruded from the capillary tubes into grooves of a mold for example, by using a plunger. Alternatively, the fibroblasts, smooth muscle cells, and endothelial cells may be incorporated separately into multicellular bodies, including for example, elongate cellular bodies. The elongate cellular bodies may contain more than one extracellular matrix component pre-mixed with the cells. The multicellular body can then be incubated at 37°C and 5% CO₂ for later incorporation into vascular tubes through the use of a bioprinter.

The multicellular bodies of the present invention may also include one or more extracellular matrix (ECM) components or one or more derivatives of one or more ECM components in addition to the plurality of cells. For example, the elongate cellular bodies may contain various ECM proteins (e.g., gelatin, fibrinogen, fibrin, collagen, fibronectin, laminin, elastin, and/or proteoglycans). The ECM components or derivatives of ECM components can be added to the cell paste used to form the elongate cellular body. The ECM components or derivatives of ECM components added to the cell paste can be purified from a human or animal source, or produced by recombinant methods known in the art. Alternatively, the ECM components or derivatives of ECM components can be naturally secreted by the cells in the elongate cellular body, or the cells used to make the elongate cellular body can be genetically manipulated by any suitable method known in the art to vary the expression level of one or more ECM components or derivatives of ECM components and/or one or more cell adhesion molecules or cell-substrate adhesion molecules (e.g., selectins, integrins, immunoglobulins, and cadherins). The ECM components or derivatives of ECM components may promote cohesion of the cells in the elongate cellular body. For example, gelatin and/or fibrinogen can suitably be added to the cell paste which is used to form the elongate cellular body. The fibrinogen can then be converted to fibrin by the addition of thrombin.

The ratio of endothelial cells to smooth muscle cells may be any ratio between 5:95 to 45:55 endothelial cells to smooth muscle cells in the multicellular body. For example, the ratio of endothelial cells to smooth muscle cells in the engineered multilayered vascular tube may be, about 10:90, about 15:85, about 20:80, about 25:75, about 30:70, about 35:65, about 40:60, or any ratio in between 5:95 to 45:55 endothelial cells to smooth muscle cells.

In some embodiments, the engineered multilayered vascular tube is substantially free of non-differentiated adult fibroblasts. In some embodiments, the engineered multilayered vascular tube is substantially free of non-differentiated adult smooth muscle cells. In some embodiments, the engineered multilayered vascular tube is substantially free of non-differentiated adult endothelial cells.

The fibroblasts of the present invention can be cultured, for example, in tissue culture flasks. When confluent, the fibroblasts can be passaged and centrifuged with supernatant removed to form a single cell paste. The fibroblast cell paste can be formed into an elongate cellular body, for example, by aspiration into a capillary tube. The elongate cellular body can then be extruded from the capillary tubes into grooves of a mold for example, by using a plunger. The elongate cellular body can then be incubated at 37°C and 5% CO₂ for later incorporation into vascular tubes through use of a bioprinter.

The smooth muscle cells of the present invention can also be cultured, for example, in tissue culture flasks. When confluent, the smooth muscle cells can be passaged and centrifuged with supernatant removed to form a single cell paste. The smooth muscle cell paste can be formed into an elongate cellular body, for example, by aspiration into a capillary tube. The elongate cellular body can then be extruded from the capillary tubes into grooves of a mold for example, by using a plunger. The elongate cellular body can then be incubated at 37°C and 5% CO₂ for later incorporation into vascular tubes through use of a bioprinter.

The endothelial cells of the present invention can also be cultured, for example, in tissue culture flasks. When confluent, the endothelial cells can be passaged and centrifuged with supernatant removed to form a single cell paste. The endothelial cell paste can be formed into an elongate cellular body, for example, by aspiration into a capillary tube. The elongate cellular body can then be extruded from the capillary tubes into grooves of a mold for example, by using a plunger. The elongate cellular body can then be incubated at 37°C and 5% CO₂ for later incorporation into vascular tubes through use of a bioprinter.

A gel matrix mold can be fabricated for incubation of the smooth muscle/endothelial and fibroblast cell cylinders. The gel matrix mold can be composed of any biocompatible gel matrix, such as agarose, agar, or other biocompatible hydrogels such as polyethylene glycol (PEG). The gel matrix material is permeable for nutrient media, but resists the in-growth, migration, and adherence of cells. The gel matrix mold can be sterilized. The gel matrix mold can be fabricated using a negative mold which is placed on top of the gel matrix and allowed to shape the gel into the form of the gel matrix mold. For example, a Teflon negative mold may be used. The elongate cellular bodies can be incubated in the gel matrix mold for 24 hours, for example, before being re-aspirated into a capillary tube for formation into a vascular structure. In some embodiments, the gel matrix mold is characterized by cylindrical shape. In some embodiments, the gel matrix mold is characterized by semi-cylindrical shape. In some embodiments, the gel matrix mold is characterized by rectangular shape.

The cells in the elongate cellular bodies can be assessed for cell viability, e.g., using routine histology, for example, by H&E staining. The cells in the elongate cellular bodies may also be assessed for ease of handling. For example, the amount the cylinders curl (e.g., curling) at the end of an incubation time point can be assessed. For example, a subjective preference scoring system can be used. For example, a curling score can be assigned to an elongate cellular body on a 1-10 point basis, where a score of 1 means the elongate cellular body exhibit no curling, and a score of 10 means that the elongate cellular body had curled to form spiral-like structures. Additionally, the integrity of elongate cellular bodies, e.g., coherence, can be assessed at the end of an incubation time point by using a subjective score. For example, these two scores can allow for selection of preferred conditions for preparing elongate cellular bodies to assure ease of handling during formation of the multilayer vascular tube. For example, a coherence score can be assigned to an elongate cellular body on a 10 point basis where a score of 1 means the cylinder maintained its smooth cylindrical structure completely, and a coherence score of 10 means that the cylindrical structure was lost.

The multilayered vascular grafts, formed through the use of a bioprinter, can be formed, for example, by assembling a plurality of multicellular bodies and allowing the bodies to cohere. In some embodiments, cells, cell aggregates, multicellular bodies, and/or layers cohere by cell-cell adhesion. In other embodiments, cells, cell aggregates, multicellular bodies, and/or layers fuse. In some embodiments, the multilayered vascular grafts are formed by assembling a plurality of elongate cellular bodies in a desired tubular shape with elastic consistency, and allowing the elongate cellular bodies to cohere. In further embodiments, the cells and/or cell aggregates in the elongate cellular bodies may cohere together to form a vascular tubular construct with elastic consistency, desired cell density, and sufficient integrity for easy manipulation and handling.

A method to produce elongate cellular bodies comprises the steps of 1) providing a cell paste containing a plurality of pre-selected cells or cell aggregates with a desired cell density and viscosity, 2) manipulating the cell paste into desired tubular shape, and 3) forming the elongate cellular bodies through maturation.

Substantially spherical cellular bodies, either alone or in combination with elongate cellular bodies, are also suitable to build a vascular tube of the type described herein. A method to produce substantially spherical cellular bodies comprises the steps of 1) providing a cell paste containing a plurality of pre-selected cells or cell aggregates with a desired cell density and viscosity, 2) manipulating the cell paste into a cylindrical shape, 3) cutting cylinders into equal fragments, 4) letting the fragments round up overnight on a gyratory shaker, and 5) forming the substantially spherical cellular bodies through maturation.

An elongate filler body can be used in combination with the aforesaid elongate cellular bodies to build a vascular tube. The elongate filler body comprises a material in a pre-determined shape, such as a rod or other mold, whereas the material is permeable for nutrient media, but prevents the in-growth, migration, and adherence of cells. The filler body unit may be made of material such as, agarose, agar, or other biocompatible hydrogels such as polyethylene glycol (PEG). During the construction of an engineered multilayer vascular tube, elongate filler bodies can be employed, according to a pre-determined pattern, to define a support structure for the vascular tube. An elongate filler body may be used to form the lumen of the multilayer vascular graft. The lumen-forming filler body may be removable in order to form the lumen of the tube. Examples of elongate filler bodies and elongate cellular bodies and methods of forming the same can be found in U.S. patent application number 12/491,228.

The engineered multilayered vascular tubes of the present invention, formed through the use of a bioprinter, in some embodiments, comprise layers. In further embodiments, the layers are characterized by the presence of one or more cell types. In still further embodiments, the layers are characterized by position within the engineered multilayered vascular tube. In some embodiments, the layers are characterized by concentration of one or more components, elements, or compounds and/or physical properties such as compliancy, strength, or elasticity. In some embodiments, one or more layers are distinct. In further embodiments, one or more layers are separable. In some embodiments, one or more layers are contiguous. In further embodiments, one or more layers are inseparable. In still further embodiments, one or more layers are characterized by properties that vary in a continuous gradient across a region of the engineered multilayered vascular tube. In some embodiments, one or more layers are characterized by properties that vary over time.

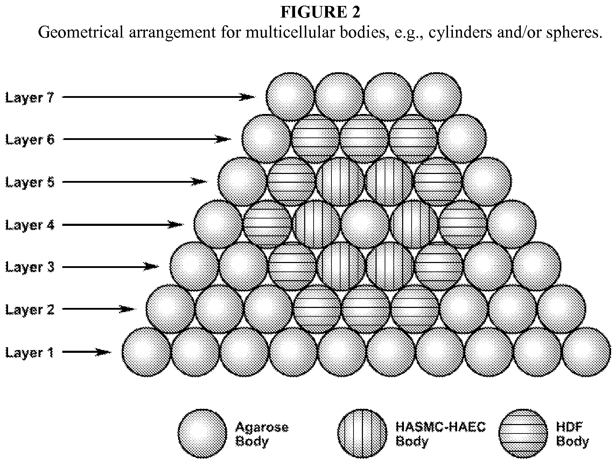

The engineered multilayered vascular, formed through the use of a bioprinter, can be fabricated using an inner layer of elongate cellular bodies of smooth muscle cells and endothelial cells, and an outer layer of elongate cellular bodies of fibroblasts. A geometrical arrangement such as that shown in Figure 2 can be used, where a geometrical pattern of elongate cellular bodies of smooth muscle cells and endothelial cells, elongate cellular bodies of fibroblasts, and elongate bodies of filler matrix can be arranged to form a three-dimensional vascular structure. The multilayered vascular tube can be formed on a base plate having a gel matrix, such as agarose. For example, a gel matrix can be aspirated into capillary tubes and gelled, e.g., in a cold (4°C) PBS solution, and extruded from the capillary and laid down straight on the gel matrix base plate to form an elongate body of filler matrix. A second elongate body of filler matrix can be adjoined to the first and the process repeated until the desired support structure is formed. A wetting solution, such as PBS, can be dispensed over the elongate bodies of filler matrix as they are deposited to keep them wet and to allow for adhesion between the bodies. Elongate bodies of fibroblasts can be deposited on the next layer above the gel matrix elongate bodies to form the outer layer of the multilayer vascular tube, and elongate bodies of smooth muscle cells and endothelial cells can further be extruded to continue forming the inner layer, on a layer-by-layer basis. After extruding each elongate cellular body, a wetting solution such as culture medium may be dispensed on top of the deposited bodies to assist in the deposition of the subsequent elongate cellular body and to prevent dehydration of the elongate cellular bodies already deposited. This process is repeated until the desired vascular structure is formed. Additionally, the engineered multilayered vascular tube can be fabricated using layers of substantially spherical cellular bodies, alone or in combination with elongate cellular bodies. The desired vascular structure may be a straight tube, or it may be a tube having one or more branches (e.g., a branched structure). Once the entire vascular construct is completed, gel matrix can be dispensed over each end of the construct and allowed to gel. Following gelation, the entire construct can be submerged in a wetting solution such as culture medium, and incubated to allow for cohesion between the cellular cylinders, e.g., for 24 hours at 37°C and 5% CO₂.

The engineered multilayered vascular tube are formed through the use of a bioprinter. For example, the engineered multilayered vascular tube may be formed by laying elongate and/or substantially spherical cellular bodies and elongate bodies of gel matrix manually, not forming part of the invention, such as with a micropipette, or automated, such as with a special-purpose bioprinter (such as the one described in U.S. patent application number 10/590,446).

Alternatively, not forming part of the invention, the multilayered vascular tubes may be formed by using a combination of various methods, such as cell seeding and assembling of a plurality of elongate cellular bodies and/or a plurality of elongate filler bodies. For example, endothelial cells may be seeded (e.g., flowed through) the lumen of a vascular tube formed of elongate cellular bodies of smooth muscle cells and elongate cellular bodies of fibroblasts. As another example, a layer of fibroblasts may be wrapped around a vascular tube formed of elongate cellular bodies of smooth muscle cells and endothelial cells. As yet another example, the pre-formed cellular body used to form the vascular tubes comprises more than one cell type. As yet another example, the layer used to form the vascular tubes comprises more than one cell type.

In some embodiments, the pre-formed cellular body used to form the vascular tubes comprises all three cell types (endothelial cells, smooth muscle cells, and fibroblasts); in such an example each cell type migrates to an appropriate position (e.g., during the time within the maturation chamber) to form the engineered multilayer vascular tube. In other embodiments, the pre-formed cellular body used to form the vascular tubes comprises two cell types (selected from endothelial cells, smooth muscle cells, and fibroblasts) and both cell types migrate to an appropriate position (e.g., during the time within the maturation chamber) to form the engineered multilayer vascular tube. In some embodiments, cells of each type are uniformly distributed within a cellular body or layer of the vascular tube. In other embodiments, cells of each type localize to particular regions within a cellular body or layer of the vascular tube.

In some embodiments, the multilayered vascular tubes of the present invention are non-innervated. In some embodiments, the multilayered vascular tubes of the present invention comprise differentiated adult fibroblasts that are non-vascular fibroblasts.

At the end of the incubation period, the surrounding gel matrix support structure may be removed and the cohered multilayer vascular tube can be placed in a maturation chamber (e.g., bioreactor) for growth, and for formation of extracellular matrix. The support structure/filler body may be removed from the lumen of the vascular tube and/or surrounding the vascular tube by pulling the filler body out, or by other methods such as thermoreversible or photosensitive methods.

Intralumenal fluid perfusion may be used during maturation of the engineered multilayered vascular tube. For example, intralumenal perfusion may be used when the multilayer vascular tube is placed in a maturation chamber (e.g., bioreactor). Intralumenal perfusion may begin at relatively low pressures and be increased during progression of maturation of the engineered multilayer tube. For example, intralumenal perfusion may be increased to levels which mimic or exceed the pressures and shear forces in native tissue. For example, internal pressures for arterial and venous multilayer tubes may be subjected to pressures of less than 60 mm Hg, 60-150 mm Hg, 150-200 mm Hg, or greater than 200 mm Hg to mimic normal and/or elevated blood pressures. Lower pressures are advisable during early stages of maturation of the engineered multilayer tube to avoid disruption of the tissue. Similarly, engineered multilayer tubes may be subjected to shear forces of less than 5 dynes/cm², 5-30 dynes/cm², 30-60 dynes/cm², or greater than 60 dynes/cm² to mimic normal and/or elevated shear forces in the circulatory system, with lower levels preferably used initially. Such techniques for intralumenal perfusion are known in the art.

Pulsatile forces may also be included in the intralumenal perfusion, as known in the art. Pulsatile forces may, for example, be used to mimic the natural pulsing of blood circulating in arteries and veins. For example, example, a pulse rate of less than 60/min, 60-90/min, 75/min, greater than 90/min, or 140-160/min, or greater than 160/min, or any other pulse rate to mimic the resting pulse of an adult or fetus can be used. The use of pulsatile force may be relative low initially, with the force increasing to physiological levels as the tissue construct more fully matures.

The engineered multilayered vascular tubes of the present invention do not utilize any pre-formed scaffold, e.g., for the formation of any layer of the vascular tube, or formation of the tubular shape. As a non-limiting example, the engineered multilayered vascular tubes of the present invention do not utilize any pre-formed, synthetic scaffolds such as polymer scaffolds, extracellular matrix layers, or any other type of pre-formed scaffold. According to the invention, the engineered multilayered vascular tube is free of any pre-formed scaffolds. In some embodiments, not forming part of the invention, the engineered multilayered vascular tube contains a detectable, but trace or trivial amount of scaffold, e.g., less than 0.5% of the total composition. In further embodiments, trace or trivial amounts of scaffold are insufficient to affect long term behavior of the graft or interfere with its primary biological function.

The engineered multilayered vascular tubes of the present invention do not utilize only the formation of sheets of cells, e.g., sheets of fibroblast cells, in order to form the vascular tube.

The engineered multilayered vascular tubes of the present invention may have an internal diameter of about 5 mm, about 4 mm, about 3 mm, about 2 mm, about 1 mm, or about 0.5 mm or smaller, or any diameter in between, preferably, about 6 mm or smaller. The engineered multilayer vascular tubes may have a length ranging from about 1 cm or shorter to 30 cm. Preferably, the vascular tube has a length ranging from about 1 cm to 30 cm.

After the engineered multilayered tubes are subject to perfusion, e.g., for about 4 days or longer, the inner layer of mixed smooth muscle cells and endothelial cells, in conjunction with the outer layer of fibroblasts, allows for cellular adhesion, reorganization and migration that allows the native structure of the intima, media, and adventitia to form, with the endothelial cells migrating to the inside, the smooth muscle cells to the middle, and the fibroblasts remain on the outer layer.

Alternatively, magnetic fields may be used to guide cellular reorganization and migration of the various cell types in the engineered multilayered tubes. For example, the engineered multilayered tubes may comprise magnetic particles (e.g., ferromagnetic nanoparticles, etc.) and subjected to magnetic fields to guide cellular reorganization and migration.

The thickness of the three layers of the vascular tubes is substantially uniform for at least a region of the length of the vascular tube, e.g., plus or minus 20% or smaller. In some embodiments, the thickness of the vascular tube is plus or minus 10% or smaller, or plus or minus 5% or smaller. The thickness of the vascular tube may also be substantially uniform over the length of the vascular tube. The thickness of each layer of the vascular tube may also resemble that of native vascular tissue.

The engineered multilayered vascular tubes of the present invention may also be compliant and of a thickness to withstand pressures comparable to native physiological blood pressures. For example, the burst strength of the engineered multilayered vascular tube may be sufficient to withstand physiological blood pressure. A variety of methods may be employed to test the biomechanical properties of engineered multilayer vascular tubes. Suitable methods for testing the biomechanical properties of a vascular tube include measurement of burst strengths and compliances. Stress-strain analyses such as single load versus elongation test, stress relaxation test, and tensile failure test are also appropriate and may be applied. Additional tests known to those of skill in the art may also be used.

The engineered multilayered vascular tubes of the present invention may be used in several applications; for example, the vascular tubes may be used in bypass grafting, for arteriovenous access, in drug testing applications, or in cardiovascular device testing applications. Examples of cardiovascular devices for which the vascular tubes may be used include stents.

Further described herein are methods for treating patients, comprising providing at least one engineered multilayered vascular tube of the present invention. A patient in need of an engineered multilayered vascular tube may be a patient in need of a coronary or peripheral bypass, or a patient in need of hemodialysis, for example. The patient in need may have damaged blood vessels. The patient in need may also be one with end-stage renal disease, diabetes, arteriosclerosis, or congenital heart birth defects, for example. The engineered multilayered vascular tubes of the present invention may be used to treat any patient in need of a replacement blood vessel.

While preferred embodiments of the present invention have been shown and described herein, it will be obvious to those skilled in the art that such embodiments are provided by way of example only.

### EXAMPLES

The following specific examples are to be construed as merely illustrative, and not limitative of the remainder of the disclosure in any way whatsoever. Without further elaboration, it is believed that one skilled in the art can, based on the description herein, utilize the present invention to its fullest extent.

### Example 1: HASMC-HAEC mixed cellular cylinders

### Materials and Methods

### 1. Cell Culture

1.1 *Smooth muscle cells:* Primary human aortic smooth muscle cells (HASMC; GIBCO/Invitrogen Corp., Carlsbad, CA) were maintained and expanded in low glucose dulbecco's modified eagle medium (DMEM; Invitrogen Corp., Carlsbad, CA) supplemented with 10% fetal bovine serum (FBS), 100 U/ml Penicillin, 0.1 mg/ml streptomycin, 0.25 µg/ml of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent cultures of HASMC's between passage 4 and 8 were used in all studies.
1.2 *Endothelial cells:* Primary human aortic endothelial cells (HAEC; GIBCO/Invitrogen Corp., Carlsbad, CA) were maintained and expanded in Medium 200 (Invitrogen Corp., Carlsbad, CA) supplemented with 2% FBS, 1 µg/ml of hydrocortisone, 10 ng/ml of human epidermal growth factor, 3 ng/ml of basic fibroblast growth factor, 10 µg/ml of heparin, 100 U/ml Penicillin, 0.1 mg/ml streptomycin, and 0.25 µg/ml of amphotericin B (all from Invitrogen Corp., Carlsbad, CA). The cells were grown on gelatin (from porcine serum; Sigma, St. Louis, MO) coated tissue culture treated flasks at 37°C and 5% CO₂. Confluent cultures of HAEC's between passage 4 and 8 were used in all studies.

### 2. Agarose mold

2.1 *Preparation of 2% w*/*v agarose solution:* 1g of low melting point agarose (Ultrapure LMP; Invitrogen Corp., Carlsbad, CA) was dissolved in 50 ml of dulbecco's phosphate buffered saline (DPBS; Invitrogen Corp., Carlsbad, CA). Briefly, the DPBS and agarose are heated to 85°C on a hot plate with constant stirring until the agarose dissolves completely. Agarose solution is sterilized by steam sterilization at 125°C for 25 minutes. The agarose solution remains in liquid phase as long as the temperature is maintained above 36.5°C. Below this temperature a phase transition occurs, the viscosity of the agarose solution increases and the agarose forms a solid gel.
2.2 *Preparation of agarose mold:* An agarose mold is fabricated for the incubation of cellular cylinders using a Teflon mold that fits a 10 cm Petri dish. Briefly, the Teflon mold is pre-sterilized using 70% ethanol solution and subjecting the mold to UV light for 45 minutes. The sterilized mold is placed on top of the 10 cm Petri dish (VWR International LLC, West Chester, PA) and securely attached. This assembly (Teflon mold + Petri dish) is maintained vertically and 45 ml of pre-warmed, sterile 2% agarose solution is poured in the space between the Teflon mold and the Petri dish. The assembly is then placed horizontally at room temperature for 1 hour to allow complete gelation of the agarose. After gelation, the Teflon print is removed and the agarose mold is washed twice using DPBS. 17.5 ml of HASMC culture medium is then added to the agarose mold.

### 3. HASMC-HAEC cylinders

3.1 *Fabrication of HASMC-HAEC mixed cellular cylinders:* To prepare mixed cellular cylinders HASMC and HAEC were individually collected and then mixed at pre-determined ratios. Briefly, the culture medium was removed from confluent culture flasks and the cells were washed with DPBS (1 ml/5 cm² of growth area). Cells were detached from the surface of the culture flasks by incubation in the presence of trypsin (1 ml/15 cm² of growth area; Invitrogen Corp., Carlsbad, CA) for 10 minutes. HASMC were detached using 0.15% trypsin while HAEC were detached using 0.1% trypsin. Following the incubation appropriate culture medium was added to the flasks (2X volume with respect to trypsin volume). The cell suspension was centrifuged at 200g for 6 minutes followed by complete removal of supernatant solution. Cell pellets were resuspended in respective culture medium and counted using a hemacytometer. Appropriate volumes of HASMC and HAEC were combined to yield mixed cell suspensions containing 5, 7.5, 10, 12.5, and 15% HAEC (as a % of total cell population). The mixed cell suspensions were centrifuged at 200g for 5 minutes followed by complete removal of supernatant solution. Mixed cell pellets were resuspended in 6 ml of HASMC culture medium and transferred to 20 ml glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 ml centrifuge tube and centrifuged at 200g for 5 minutes. After removal of the supernatant medium, the cell pellet was resuspended in 400 µl of HASMC culture medium and pipetted up and down several times to ensure all cell clusters were broken. The cell suspension was transferred into a 0.5 ml microfuge tube (VWR International LLC, West Chester, PA) placed inside a 15 ml centrifuge tube followed by centrifugation at 2000g for 4 minutes to form a highly dense and compact cell pellet. The supernatant medium was aspirated and the cells were transferred into capillary tubes (OD 1.5 mm, ID 0.5 mm, L 75 mm; Drummond Scientific Co., Broomall, PA) by aspiration so as to yield cell cylinders 50 mm in length. The cell paste inside the capillaries were incubated in HASMC medium for 20 minutes at 37°C and 5% CO₂. The cellular cylinders were then extruded from the capillary tubes into the grooves of the agarose mold (covered with HASMC medium) using the plunger supplied with the capillaries. The cellular cylinders were incubated for 24 and 48 hours at 37°C and 5% CO₂.

### 4. Evaluation of mixed cell cylinder viability and ease of handling

4.1 *Assessment of cell viability:* In order to assess cell viability in mixed cell cylinders at the end of 24, 48 and 72 hours incubation routine histology was performed. Briefly, at each time point, the mixed cell cylinders were aspirated back into the capillary tubes (manually using the plunger) and transferred to multi-well plates. The cylinders were fixed using 10% neutral buffered formalin for at least 24 hours before being paraffin embedded. Embedded cylinders were sectioned (crosswise) and H & E staining was performed.
4.2 *Assessment of ease of handling:* In order to assess ease of handling of mixed cell cylinders two separate parameters were evaluated. First, the amount the cylinders curled at the end of each incubation time point was assessed. A subjective preference scoring system was used. Second, the integrity of the cell cylinders was also assessed at the end of each incubation time point using a second subjective score. Combined, these two scores allowed selection of preferred conditions for preparing cylinders to assure ease of handling during later steps.

### Results

### 1. Cell viability and ease of handling of HASMC-HAEC mixed cell cylinders

HASMC-HAEC mixed cell cylinders were fabricated so as to contain 5, 7.5, 10, 12.5 and 15% HAEC as per the method described above. Cylinders were incubated in the agarose mold for 24 and 48 hours. At each time point, the cylinders were assigned a curling and coherence score. Tables 1 and 2 summarize the results. As can be seen, as incubation time increases from 24 to 48 hours the ease of handling decreases for all mixed cell cylinders. Cylinders that contain 15% HAEC and remainder 85%-HASMC are easiest to handle after 24 hours of incubation period in the agarose mold. Furthermore, cylinders containing 5% HAEC and 95% HASMC are most difficult to handle at both time points.

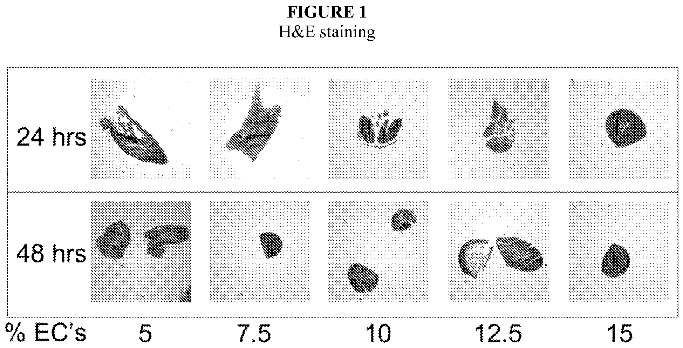

H&E staining (Fig. 1) was utilized to assess for cell viability for each condition at all time points. Staining revealed that all cylinders incubated for 24 hours in the agarose mold contain cells that are most viable (as evidenced by least amount of pink staining) and cell viability decreases with increasing incubation time. Furthermore, after 24 hours of incubation in the agarose mold, cell viability in cylinders containing 15% HAEC and 85% HASMC was the highest.

A "curling score" was assigned to each cylinder on a 10-point basis where a score of 1 meant the cylinders exhibited no curling and a score of 10 meant that cylinders had curled to form spiral like structures.

A "coherence score" was assigned to each cylinder on a 10-point basis where a score of 1 meant the cylinders maintained their smooth cylindrical structure completely and a coherence score of 10 meant that the cylindrical structure was completely lost.

**Table 1: Curling scores**

| **Incubation time (Hours)** | **% HAEC (% of total number of cells)** | | | | |
|---|---|---|---|---|---|
| | 5 | 7.5 | 10 | 12.5 | 15 |
| 24 | 3 | 2 | 3 | 2 | 1 |
| 48 | 5 | 4 | 5 | 5 | 3 |

**Table 2: Coherence scores**

| **Incubation time (Hours)** | **% HAEC (% of total number of cells)** | | | | |
|---|---|---|---|---|---|
| | 5 | 7.5 | 10 | 12.5 | 15 |
| 24 | 4 | 3 | 3 | 2 | 1 |
| 48 | 7 | 6 | 6 | 6 | 5 |

### Example 2: Multi-layered vascular tubes

### Materials and Methods

### 1. Cell Culture

1.1 *Smooth muscle cells:* Primary human aortic smooth muscle cells (HASMC; GIBCO/Invitrogen Corp., Carlsbad, CA) were maintained and expanded in low glucose dulbecco's modified eagle medium (DMEM; Invitrogen Corp., Carlsbad, CA) supplemented with 10% fetal bovine serum (FBS), 100 U/ml Penicillin, 0.1 mg/ml streptomycin, 0.25 µg/ml of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent cultures of HASMC between passage 4 and 8 were used in all studies.
1.2 *Endothelial cells:* Primary human aortic endothelial cells (HAEC; GIBCO/Invitrogen Corp., Carlsbad, CA) were maintained and expanded in Medium 200 (Invitrogen Corp., Carlsbad, CA) supplemented with 2% FBS, 1 µg/ml of hydrocortisone, 10 ng/ml of human epidermal growth factor, 3 ng/ml of basic fibroblast growth factor, 10 µg/ml of heparin, 100 U/ml Penicillin, 0.1 mg/ml streptomycin, and 0.25 µg/ml of amphotericin B (all from Invitrogen Corp., Carlsbad, CA). The cells were grown on gelatin (from porcine serum; Sigma, St. Louis, MO) coated tissue culture treated flasks at 37°C and 5% CO₂. Confluent cultures of HAEC between passage 4 and 8 were used in all studies.
1.3 *Fibroblasts:* Primary human dermal fibroblasts (HDF; GIBCO/Invitrogen Corp., Carlsbad, CA) were maintained and expanded in Medium 106 (Invitrogen Corp., Carlsbad, CA) supplemented with 2% FBS, 1 µg/ml of hydrocortisone, 10 ng/ml of human epidermal growth factor, 3 ng/ml of basic fibroblast growth factor, 10 µg/ml of heparin, 100 U/ml Penicillin, and 0.1 mg/ml streptomycin (all from Invitrogen Corp., Carlsbad, CA) at 37°C and 5% CO₂. Confluent cultures of HDF between passage 4 and 8 were used in all studies.

### 2. Agarose solutions and mold

2.1 *Preparation of 2% and 4% (w*/*v) agarose solution:* 1g or 2g (for 2% or 4% respectively) of low melting point agarose (Ultrapure LMP; Invitrogen Corp., Carlsbad, CA) was dissolved in 50 ml of dulbecco's phosphate buffered saline (DPBS; Invitrogen Corp., Carlsbad, CA). Briefly, the DPBS and agarose are heated to 85°C on a hot plate with constant stirring until the agarose dissolves completely. Agarose solution is sterilized by steam sterilization at 125°C for 25 minutes. The agarose solution remains in liquid phase as long as the temperature is maintained above 36.5°C. Below this temperature a phase transition occurs, the viscosity of the agarose solution increases and the agarose forms a solid gel.
2.2 *Preparation of agarose mold:* An agarose mold was fabricated for the incubation of cellular cylinders using a Teflon mold that fit a 10 cm Petri dish. Briefly, the Teflon mold was pre-sterilized using 70% ethanol solution and subjecting the mold to UV light for 45 minutes. The sterilized mold was placed on top of the 10 cm Petri dish (VWR International LLC, West Chester, PA) and securely attached. This assembly (Teflon mold + Petri dish) was maintained vertically and 45 ml of pre-warmed, sterile 2% agarose solution was poured in the space between the Teflon mold and the Petri dish. The assembly was then placed horizontally at room temperature for 1 hour to allow complete gelation of the agarose. After gelation, the Teflon print was removed and the agarose mold was washed twice using DPBS. Then, either 17.5 ml of HASMC culture medium is added to the agarose mold for incubating HASMC-HAEC mixed cell cylinders or 17.5ml of HDF culture medium is added to the agarose mold for incubating HDF cell cylinders.

### 3. Cellular cylinders

3.1 *Fabrication of HASMC-HAEC mixed cellular cylinders:* To prepare mixed cellular cylinders HASMC and HAEC were individually collected and then mixed at pre-determined ratios. Briefly, the culture medium was removed from confluent culture flasks and the cells were washed with DPBS (1 ml/5 cm² of growth area). Cells were detached from the surface of the culture flasks by incubation in the presence of trypsin (1 ml/15 cm² of growth area; Invitrogen Corp., Carlsbad, CA) for 10 minutes. HASMC were detached using 0.15% trypsin while HAEC were detached using 0.1% trypsin. Following the incubation appropriate culture medium was added to the flasks (2X volume with respect to trypsin volume). The cell suspension was centrifuged at 200g for 6 minutes followed by complete removal of supernatant solution. Cell pellets were resuspended in respective culture medium and counted using a hemacytometer. Appropriate volumes of HASMC and HAEC were combined to yield a mixed cell suspension containing 15% HAEC and remainder 85% HASMC (as a % of total cell population). The mixed cell suspension was centrifuged at 200g for 5 minutes followed by complete removal of supernatant solution. Mixed cell pellets were resuspended in 6 ml of HASMC culture medium and transferred to 20 ml glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 ml centrifuge tube and centrifuged at 200g for 5 minutes. After removal of the supernatant medium, the cell pellet was resuspended in 400 µl of HASMC culture medium and pipetted up and down several times to ensure all cell clusters were broken. The cell suspension was transferred into a 0.5 ml microfuge tube (VWR International LLC, West Chester, PA) placed inside a 15 ml centrifuge tube followed by centrifugation at 2000g for 4 minutes to form a highly dense and compact cell pellet. The supernatant medium was aspirated and the cells were transferred into capillary tubes (OD 1.5 mm, ID 0.5 mm, L 75 mm; Drummond Scientific Co., Broomall, PA) by aspiration so as to yield cell cylinders 50 mm in length. The cell paste inside the capillaries was incubated in HASMC medium for 20 minutes at 37°C and 5% CO₂. The cellular cylinders were then extruded from the capillary tubes into the grooves of the agarose mold (covered with HASMC medium) using the plunger supplied with the capillaries. The cellular cylinders were incubated for 24 hours at 37°C and 5% CO₂.
3.2 *Fabrication of HDF cell cylinders:* HDF cylinders were prepared using a method similar to preparing HASMC-HAEC mixed cellular cylinders. Briefly, the culture medium was removed from confluent HDF culture flasks and the cells were washed with DPBS (1 ml/5 cm² of growth area). Cells were detached from the surface of the culture flasks by incubation in the presence of trypsin (0.1%; 1 ml/15 cm² of growth area; Invitrogen Corp., Carlsbad, CA) for 10 minutes. Following the incubation HDF culture medium was added to the flasks (2X volume with respect to trypsin volume). The cell suspension was centrifuged at 200g for 6 minutes followed by complete removal of supernatant solution. Cell pellets were resuspended in 6 ml of HDF culture medium and transferred to 20 ml glass vials (VWR International LLC, West Chester, PA), followed by incubation on a orbital shaker at 150 rpm for 75 minutes, and at 37°C and 5% CO₂. Post-incubation, the cell suspension was transferred to a 15 ml centrifuge tube and centrifuged at 200g for 5 minutes. After removal of the supernatant medium, the cell pellet was resuspended in 400 µl of HDF culture medium and pipetted up and down several times to ensure all cell clusters were broken. The cell suspension was transferred into a 0.5 ml microfuge tube (VWR International LLC, West Chester, PA) placed inside a 15 ml centrifuge tube followed by centrifugation at 2000g for 4 minutes to form a highly dense and compact cell pellet. The supernatant medium was aspirated and the cells were transferred into capillary tubes (OD 1.5 mm, ID 0.5 mm, L 75 mm; Drummond Scientific Co., Broomall, PA) by aspiration so as to yield cell cylinders 50 mm in length. The cell paste inside the capillaries were incubated in HDF culture medium for 20 minutes at 37°C and 5% CO₂. The cellular cylinders were then extruded from the capillary tubes into the grooves of the agarose mold (covered with HDF medium). The cellular cylinders were incubated for 24 hours at 37°C and 5% CO₂.

### 4. Fabrication of multi-layered vascular tubes

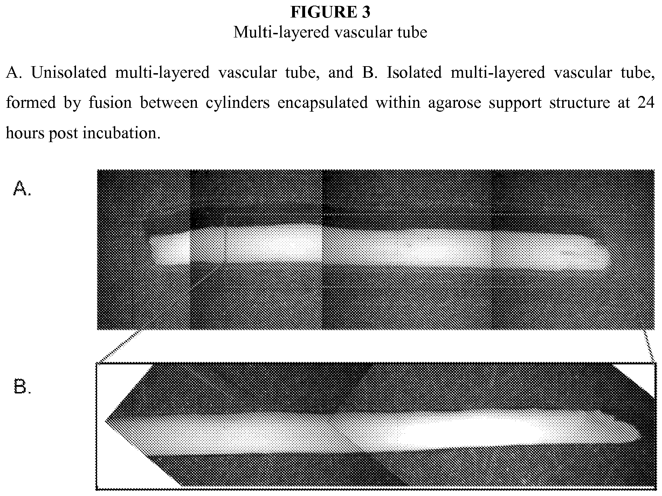

4.1 *Preparation of agarose base plate:* An agarose base plate was fabricated by dispensing 10 ml of pre-warmed (> 40°C) agarose (2% w/v) into a 10 cm Petri dish. Immediately after dispensing, the agarose is evenly spread so as to cover the entire base of the dish and form a uniform layer. The Petri dish is incubated at room temperature for 20 minutes to allow the agarose to gel completely.
4.2 *Multi-layered vascular tube:* Vascular tubes consisting of an outer layer of HDF and an inner layer of HASMC-HAEC were fabricated utilizing HDF cylinders, and HASMC-HAEC mixed cell cylinders. A geometrical arrangement as shown in Figure 2 was utilized. Briefly, at the end of the 24-hour incubation period mature HDF and HASMC-HAEC cylinders were aspirated back into the capillary tubes and placed in appropriate culture medium until further use. The support structure consisting of agarose rods was prepared as follows. Pre-warmed 2% agarose was aspirated into the capillary tubes (L=50 mm) and rapidly cooled in cold PBS solution (4°C). The 5 cm long gelled agarose cylinder was extruded from the capillary (using the plunger) and laid down straight on the agarose base plate. A second agarose cylinder was adjoined to the first one and the process was repeated until 10 agarose cylinders were deposited to form the first layer. At this point 20 µl of PBS was dispensed above the agarose cylinders to keep them wet. Further six agarose cylinders were deposited on top of layer 1 at positions as shown in Figure 2 (layer 2). Three HDF cylinders were then deposited at positions 4, 5 and 6 to complete layer 2. After extruding each HDF cylinder 40 µl of HDF culture medium was dispensed on top of the deposited cylinder to assist the deposition of the subsequent cylinder as well as to prevent dehydration of the cellular cylinders. Next agarose cylinders for layer 3 were deposited followed by HDF cylinders at positions 3 and 6. Following rewetting of the structure with HDF culture medium, HASMC-HAEC mixed cylinders were laid down in positions 4 and 5. Subsequently, 40 µl of HASMC medium and 40 µl of HDF medium were dispensed on top of the cell cylinders. Layer 4 was completed by depositing agarose cylinders at positions 1 and 7, HDF cylinders at positions 2 and 6, HASMC-HAEC mixed cylinders at positions 3 and 5, and finally a 4% agarose cylinder at position 4. Layers 5, 6 and 7 were completed similarly by laying down agarose cylinders followed by HDF cylinders and finally HASMC-HAEC cylinders at positions shown in Figure 2. Once the entire construct was completed 0.5 ml of warm agarose was dispensed over each end of the construct and allowed to gel at room temperature for 5 minutes. Following gelation of that agarose, 30 ml of HASMC medium was added to the Petri dish (to ensure the entire construct was completely submerged). The construct was incubated for 24 hours at 37°C and 5% CO₂ to allow for cohesion between the cellular cylinders. At the end of 24 hours, the surrounding agarose support structure was removed and the cohered multi-layered vascular tube was obtained (Fig. 3).

### Example 3: Blood Vessels Constructs with cells from the SVF of adipose tissue

### Materials and Methods

### 1. Cell Culture

1.1 *SMC-like cells from the SVF:* SMC-like cells were generated from the adherent fraction of cells isolated after collagenase digestion of lipoaspirates. This digestion produces a population of cells known as the stromal vascular fraction (SVF). The cells of the SVF can be plated on standard tissue culture plastic and adherent cells can be further selected with appropriate culture conditions. SMC-like cells from the SVF of adipose tissue lipoaspirates were maintained and expanded in high glucose dulbecco's modified eagle medium (DMEM; Invitrogen Corp., Carlsbad, CA) supplemented with 10% fetal bovine serum (FBS), 100 U/mL Penicillin, 0.1 mg/ml streptomycin, 0.25 µg/ml of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent subcultures of SVF-SMC between passage 3 and 8 were used in all studies.
1.2 *SVF-EC*: Endothelial cells from the stromal vascular fraction (SVF) were maintained and expanded in growth media that is commonly used to grow primary isolates of *bona fide* endothelial cells (EC). Specifically, SVF-EC were maintained in M199 supplemented with 10% FBS, 1µg/ml of hydrocortisone, 10 ng/ml of human epidermal growth factor, 3 ng/ml basic fibroblast growth factor, 10 µg/ml of heparin, 100 U/ml Penicillin, and 0.1 mg/ml streptomycin. The cells were grown on tissue culture-treated flasks at 37°C and 5% CO₂. Confluent cultures of SVF-EC between passage 3 and 8 were used in all studies.

### 2. Agarose solutions and mold

2.1 *Preparation of 2% and 4% (w*/*v) agarose solution:* 1g or 2g (for 2% or 4% respectively) of low melting point agarose (Ultrapure LMP; Invitrogen Corp., Carlsbad, CA) was dissolved in 50 ml of dulbecco's phosphate buffered saline (DPBS; Invitrogen Corp., Carlsbad, CA). Briefly, the DPBS and agarose are heated to 85°C on a hot plate with constant stirring until the agarose dissolves completely. Agarose solution is sterilized by steam sterilization at 125°C for 25 minutes. The agarose solution remains in liquid phase as long as the temperature is maintained above 36.5°C. Below this temperature a phase transition occurs, the viscosity of the agarose solution increases and the agarose forms a solid gel.
2.2 *Preparation of agarose mold:* An agarose mold was fabricated for the incubation of cellular cylinders using a Teflon mold that fit a 100 mm Petri dish. Briefly, the Teflon mold was pre-sterilized using 70% ethanol solution and subjecting the mold to UV light for 45 minutes. The sterilized mold was placed on top of the 100 mm Petri dish (VWR International LLC, West Chester, PA) and securely attached. This assembly (Teflon mold + Petri dish) was maintained vertically and 45 ml of pre-warmed, sterile 2% agarose solution was poured in the space between the Teflon mold and the Petri dish. The assembly was then placed horizontally at room temperature for 1 hour to allow complete gelation of the agarose. After gelation, the Teflon print was removed and the agarose mold was washed twice using DPBS. Then, either 17.5 ml of HASMC culture medium is added to the agarose mold for incubating SVF-SMC:SVF-EC mixed cell cylinders or 17.5 ml of HDF culture medium is added to the agarose mold for incubating HDF cell cylinders.

### 3. Cellular cylinders

3.1 *Fabrication of SVF-SMC:SVF-EC mixed cellular cylinders:* To prepare mixed cellular cylinders SVF-SMC and SVF-EC were individually collected and then mixed at pre-determined ratios. Briefly, the culture medium was removed from confluent culture flasks and the cells were washed with DPBS (1 ml/5 cm² of growth area). Cells were detached from the surface of the culture flasks by incubation in the presence of TrypLE (Invitrogen Corp., Carlsbad, CA) for 5 to 10 minutes. Following the incubation appropriate culture medium was added to the flasks to quench enzyme activity. The cell suspension was centrifuged at 200g for 6 minutes followed by complete removal of supernatant solution. Cell pellets were resuspended in respective culture medium and counted using a hemacytometer. Appropriate volumes of SVF-SMC and SVF-EC were combined to yield a mixed cell suspension containing 15% SVF-EC and remainder 85% SVF-SMC (as a % of total cell population). The mixed cell suspension was centrifuged at 200g for 5 minutes followed by complete removal of supernatant solution. Mixed cell pellets were resuspended in 6 ml of SVF-SMC culture medium and transferred to 20 ml glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 ml centrifuge tube and centrifuged at 200g for 5 minutes. After removal of the supernatant medium, the cell pellet was resuspended in 400 µl of SVF-SMC culture medium and pipetted up and down several times to ensure all cell clusters were broken. The cell suspension was transferred into a 0.5 ml microfuge tube (VWR International LLC, West Chester, PA) placed inside a 15 ml centrifuge tube followed by centrifugation at 2000g for 4 minutes to form a highly dense and compact cell pellet. The supernatant medium was aspirated and the cells were transferred into capillary tubes (OD 1.25 mm, ID 0.266 mm, L 75 mm; Drummond Scientific Co., Broomall, PA) by aspiration so as to yield cell cylinders 50 mm in length. The cell paste inside the capillaries was incubated in SVF-SMC medium for 20 minutes at 37°C and 5% CO₂. The cellular cylinders were then extruded from the capillary tubes into the grooves of the agarose mold (covered with SVF-SMC medium) using the plunger supplied with the capillaries. The cellular cylinders were incubated for 6 to 12 hours at 37°C and 5% CO₂.

### 4. Fabrication of vascular tubes

4.1 *Preparation of agarose base plate:* An agarose base plate was fabricated by dispensing 12 ml of pre-warmed (> 60°C) agarose (2% w/v) into a 10 cm Petri dish. Immediately after dispensing, the agarose is evenly spread so as to cover the entire base of the dish and form a uniform layer. The Petri dish is incubated at room temperature for 20 minutes to allow the agarose to gel completely.
4.2 *Vascular tube:* Vascular tubes consisting of SVF-SMC:SVF-EC mixed cell cylinders were fabricated. Briefly, at the end of the 6-hour incubation period mature SVF-SMC and SVF-EC cylinders were aspirated back into the capillary tubes and placed in appropriate culture medium until further use. The support structure consisting of agarose rods was prepared as follows. Pre-warmed 2% agarose was aspirated into the capillary tubes (L=50 mm) and rapidly cooled in cold PBS solution (4°C). The 5 cm long gelled agarose cylinder was extruded from the capillary (using the plunger) and laid down straight on the agarose base plate. A second agarose cylinder was adjoined to the first one and the process was repeated until an appropriate number of agarose cylinders had been extruded to accommodate the final tubular geometry. Tubular vessel constructs containing SVF-SMC and SVF-EC multicellular cylinders were fabricated using a layer-by-layer printing process, where the agarose cylinders in the center of the tubular cellular construct were comprised of 4% agarose. Once the entire construct was completed 0.5 ml of warm agarose was dispensed over each end of the construct and allowed to gel at room temperature for 5 minutes. Following gelation of that agarose, 30 ml of SVF-SMC medium was added to the Petri dish (to ensure the entire construct was completely submerged). The construct was incubated for 12 to 24 hours at 37°C and 5% CO₂ to allow for cohesion between adjacent cellular cylinders. At the end of 12 to 24 hours, the surrounding agarose support structure was removed and the cohered vascular tube was isolated.

### Example 4. Blood vessel constructs with HASMC-HDF-HAEC multicellular cylinders.

### Materials and Methods

### 1. Cell Culture

1.1 *Smooth muscle cells:* Primary human aortic smooth muscle cells (HASMC; GIBCO/Invitrogen Corp., Carlsbad, CA) were maintained and expanded in low glucose dulbecco's modified eagle medium (DMEM; Invitrogen Corp., Carlsbad, CA) supplemented with 10% fetal bovine serum (FBS), 100 U/ml Penicillin, 0.1 mg/ml streptomycin, 0.25 µg/ml of amphotericin B, 0.01M of HEPES (all from Invitrogen Corp., Carlsbad, CA), 50 mg/L of proline, 50 mg/L of glycine, 20 mg/L of alanine, 50 mg/L of ascorbic acid, and 3 µg/L of CuSO₄ (all from Sigma, St. Louis, MO) at 37°C and 5% CO₂. Confluent cultures of HASMC between passage 4 and 8 were used in all studies.
1.2 *Endothelial cells:* Primary human aortic endothelial cells (HAEC; GIBCO/Invitrogen Corp., Carlsbad, CA) were maintained and expanded in Medium 199 (Invitrogen Corp., Carlsbad, CA) supplemented with 10% FBS, 1 µg/ml of hydrocortisone, 10 ng/ml of human epidermal growth factor, 3 ng/ml of basic fibroblast growth factor, 10 µg/ml of heparin, 100 U/ml Penicillin, 0.1 mg/ml streptomycin, and 0.25 µg/ml of amphotericin B (all from Invitrogen Corp., Carlsbad, CA). The cells were grown on gelatin (from porcine serum; Sigma, St. Louis, MO) coated tissue culture treated flasks at 37°C and 5% CO₂. Confluent cultures of HAEC between passage 4 and 8 were used in all studies.
1.3 *Fibroblasts:* Primary human dermal fibroblasts (HDF; GIBCO/Invitrogen Corp., Carlsbad, CA) were maintained and expanded in Medium 106 (Invitrogen Corp., Carlsbad, CA) supplemented with 2% FBS, 1 µg/ml of hydrocortisone, 10 ng/ml of human epidermal growth factor, 3 ng/ml of basic fibroblast growth factor, 10 µg/ml of heparin, 100 U/ml Penicillin, and 0.1 mg/ml streptomycin (all from Invitrogen Corp., Carlsbad, CA) at 37°C and 5% CO₂. Confluent cultures of HDF between passage 4 and 8 were used in all studies.

### 2. Agarose solutions and mold

2.1 *Preparation of 2% and 4% (w*/*v) agarose solution:* 1g or 2g (for 2% or 4% respectively) of low melting point agarose (Ultrapure LMP; Invitrogen Corp., Carlsbad, CA) was dissolved in 50 ml of dulbecco's phosphate buffered saline (DPBS; Invitrogen Corp., Carlsbad, CA). Briefly, the DPBS and agarose are heated to 85°C on a hot plate with constant stirring until the agarose dissolves completely. Agarose solution is sterilized by steam sterilization at 125°C for 25 minutes. The agarose solution remains in liquid phase as long as the temperature is maintained above 36.5°C. Below this temperature a phase transition occurs, the viscosity of the agarose solution increases and the agarose forms a solid gel.
2.2 *Preparation of agarose mold:* An agarose mold was fabricated for the incubation of cellular cylinders using a Teflon mold that fit a 10 cm Petri dish. Briefly, the Teflon mold was pre-sterilized using 70% ethanol solution and subjecting the mold to UV light for 45 minutes. The sterilized mold was placed on top of the 10 cm Petri dish (VWR International LLC, West Chester, PA) and securely attached. This assembly (Teflon mold + Petri dish) was maintained vertically and 45 ml of pre-warmed, sterile 2% agarose solution was poured in the space between the Teflon mold and the Petri dish. The assembly was then placed horizontally at room temperature for 1 hour to allow complete gelation of the agarose. After gelation, the Teflon print was removed and the agarose mold was washed twice using DPBS. Then 17.5 ml of HASMC culture medium was added to the agarose mold for incubating the mixed cell cylinders.

### 3. HASMC-HDF-HAEC cylinders

3.1 *Fabrication of HASMC-HDF-HAEC mixed cellular cylinders:* To prepare mixed cellular cylinders HASMC, HDF, HAEC were individually collected and then mixed at pre-determined ratios (e.g., HASMC:HDF:HAEC ratios of 70:25:5). Briefly, the culture medium was removed from confluent culture flasks and the cells were washed with DPBS (1 ml/10 cm² of growth area). Cells were detached from the surface of the culture flasks by incubation in the presence of trypsin (1 ml/15 cm² of growth area; Invitrogen Corp., Carlsbad, CA) for 10 minutes. HASMC and HDF were detached using 0.15% trypsin while HAEC were detached using 0.1% trypsin. Following the incubation appropriate culture medium was added to the flasks (2X volume with respect to trypsin volume). The cell suspension was centrifuged at 200g for 6 minutes followed by complete removal of supernatant solution. Cell pellets were resuspended in respective culture medium and counted using a hemacytometer. Appropriate volumes of HASMC, HDF and HAEC were combined to yield mixed cell suspensions. The mixed cell suspensions were centrifuged at 200g for 5 minutes followed by aspiration of the supernatant solution. Mixed cell pellets were resuspended in 6 ml of HASMC culture medium and transferred to 20 ml glass vials (VWR International LLC, West Chester, PA), followed by incubation on a orbital shaker at 150 rpm for 60 minutes, and at 37°C and 5% CO₂. This allows the cells to aggregate with one another and initiate cell-cell adhesions. Post-incubation, the cell suspension was transferred to a 15 ml centrifuge tube and centrifuged at 200g for 5 minutes. After removal of the supernatant medium, the cell pellet was resuspended in 400 µl of HASMC culture medium and pipetted up and down several times to ensure all cell clusters were broken. The cell suspension was transferred into a 0.5 ml microfuge tube (VWR International LLC, West Chester, PA) placed inside a 15 ml centrifuge tube followed by centrifugation at 2000g for 4 minutes to form a highly dense and compact cell pellet. The supernatant medium was aspirated and the cells were transferred into capillary tubes (OD 1.25 mm, ID 0.266 mm, L 75 mm; Drummond Scientific Co., Broomall, PA) by aspiration so as to yield cell cylinders 50 mm in length. The cell paste inside the capillaries was incubated in HASMC medium for 20 minutes at 37°C and 5% CO₂. The cellular cylinders were then extruded from the capillary tubes into the grooves of the agarose mold (covered with HASMC medium) using the plunger supplied with the capillaries. The cellular cylinders were incubated for 6 to 24 hours at 37°C and 5% CO₂.

### 4. Fabrication of vascular tubes with multicellular cylinders (HASMC:HDF:HAEC)

4.1 *Preparation of agarose base plate:* An agarose base plate was fabricated by dispensing 10 ml of pre-warmed (> 40°C) agarose (2% w/v) into a 10 cm Petri dish. Immediately after dispensing, the agarose is evenly spread so as to cover the entire base of the dish and form a uniform layer. The Petri dish is incubated at room temperature for 20 minutes to allow the agarose to gel completely.
4.2 *Multicellular vascular tubes:* Vascular tubes consisting of cylinders containing all three cell types, HASMC:HDF:HAEC were fabricated utilizing multicellular cylinders. A geometrical arrangement with 6 or 12 cellular cylinders and 1 or 7 central agarose cylinders, respectively, was utilized. Briefly, at the end of the 6- to 12-hour incubation period mature HASMC-HDF-HAEC cylinders were aspirated back into the capillary tubes and placed in appropriate culture medium until further use. The support structure consisting of agarose rods was prepared as follows. Pre-warmed 2% agarose was aspirated into the capillary tubes (L=50 mm) and rapidly cooled in cold PBS solution (4°C). The 5 cm long gelled agarose cylinder was extruded from the capillary (using the plunger) and laid down straight on the agarose base plate. A second agarose cylinder was adjoined to the first one and the process was repeated until 7 or 10 agarose cylinders were deposited to form the first layer. Described in further detail is the fabrication of a vascular tube with 6 multicellular cylinders and 1 central agarose cylinder. At this point 20 µl of PBS was dispensed above the agarose cylinders to keep them hydrated. Further two agarose cylinders were deposited on top of layer 1, with the first agarose cylinder deposited at the leftmost edge and the second agarose cylinder to the right of that. Two multicellular HASMC-HDF-HAEC cylinders were then deposited to the right of the two agarose cylinders in Layer 2. Subsequently, 20 µl of HASMC medium was dispensed on top of the cell cylinders. Two more agarose cylinders were then deposited to the right of the two multicellular cylinders. Layer 3 was constructed with an agarose cylinder at the leftmost edge of the layer. A multicellular cylinder was deposited to the right of that cylinder. A 4% agarose cylinder was then deposited to the right of the multicellular cylinder. Another multicellular cylinder was deposited to the right of the 4% agarose cylinder. Finally, another 2% agarose cylinder was deposited to the right of the second multicellular cylinder. Subsequently, 20 µl of HASMC medium was dispensed on top of the cell cylinders. Layer 4 was begun with a 2% agarose cylinder at the rightmost edge, followed by deposition of two cellular cylinders to the right of the agarose cylinder. Layer 4 was finished with a final agarose cylinder to the right of the two multicellular cylinders. Subsequently, 20 µl of HASMC medium was dispensed on top of the cell cylinders. Layer 5 was constructed by depositing 3 agarose cylinders on top of the Layer 4. Once the entire construct was completed 0.5 ml of warm agarose was dispensed over each end of the construct and allowed to gel at room temperature for 5 minutes. Following gelation of that agarose, 30 ml of HASMC medium was added to the Petri dish (to ensure the entire construct was completely submerged). The construct was incubated for 12 to 24 hours at 37°C and 5% CO₂ to allow for cohesion between adjacent cellular cylinders. At the end of 12 to 24 hours, the surrounding agarose support structure was removed and the cohered multi-layered vascular tube was obtained (Fig. 3).

## Claims

1. An engineered multilayered vascular tube formed through the use of a bioprinter comprising at least one layer of differentiated adult fibroblasts and at least one layer of differentiated adult smooth muscle cells, wherein either the at least one layer of differentiated adult fibroblasts or the at least one layer of differentiated smooth muscle cells further comprises differentiated adult endothelial cells, wherein said engineered multilayered vascular tube has a ratio of endothelial cells to smooth muscle cells of 5:95 to 45:55, and wherein said engineered multilayered vascular tube has one, two, three, or all of the following features: (a) the engineered multilayered vascular tube is compliant; (b) the internal diameter of the engineered multilayered vascular tube is 6 mm or smaller; (c) the length of the engineered multilayered vascular tube is up to 30 cm; and (d) the thickness of the engineered multilayered vascular tube is substantially uniform along a region of the tube; provided that the engineered multilayered vascular tube is free of any pre-formed scaffold.

2. The engineered multilayered vascular tube of claim 1 comprising an outer layer of differentiated adult fibroblasts, and at least one inner layer of differentiated adult smooth muscle cells and differentiated adult endothelial cells.

3. The engineered multilayered vascular tube of claim 1 or 2, wherein the burst strength is sufficient to withstand physiological blood pressure.

4. The engineered multilayered vascular tube of any one of claims 1 to 3, wherein the ratio of endothelial cells to smooth muscle cells is 15:85.

5. The engineered multilayered vascular tube of any preceding claim, wherein the internal diameter of the engineered multilayered vascular tube is 0.5 mm or smaller.

6. The engineered multilayered vascular tube of any preceding claim, wherein the thickness of the engineered multilayered vascular tube is substantially uniform over the length of the tube.

7. The engineered multilayered vascular tube of any preceding claim, wherein the engineered multilayered vascular tube is substantially free of non-differentiated adult fibroblasts, non-differentiated adult smooth muscle cells, and/or non-differentiated adult endothelial cells, and/or wherein the tube has a branched structure.

8. The engineered multilayered vascular tube of any preceding claim, wherein the tube is non-innervated, and/or wherein said differentiated adult fibroblasts are non-vascular fibroblasts.

9. The engineered multilayered vascular tube of claim 3, for use in
a) drug testing or cardiovascular device testing, optionally wherein the cardiovascular device is a stent; or
b) repairing damaged blood vessels; or
c) treating a patient in need of a coronary or peripheral bypass or arteriovenous access; or
d) treating a patient with end-stage renal disease, diabetes, arteriosclerosis, or congenital heart birth defects.

10. The engineered multilayered vascular tube of claim 9, wherein the fibroblasts, smooth muscle cells, and/or endothelial cells of the engineered multilayered vascular tube are autologous.

11. A method of forming an engineered multilayered vascular tube, comprising:
positioning at least one body of filler matrix, at least one multicellular body of smooth muscle cells wherein the cells are cohered to one another, at least one multicellular body of endothelial cells wherein the cells are cohered to one another, and at least one multicellular body of fibroblast cells wherein the cells are cohered to one another to form a desired engineered multilayered vascular tube structure, wherein the ratio of endothelial cells to smooth muscle cells in the engineered multilayered vascular tube is 5:95 to 45:55, and wherein one or more filler matrix bodies form the lumen of the engineered multilayered vascular tube, and a plurality of filler matrix bodies surround the engineered multilayered vascular tube; and allowing the multicellular bodies of the engineered multilayer vascular tube to cohere; provided that no pre-formed scaffold is used at any time, and provided that the engineered multilayered vascular tube is formed through use of a bioprinter.

12. The method of claim 11, wherein the diameter of the lumen of the engineered multilayered vascular tube is 6 mm or smaller.

13. The method of claim 11, further comprising culturing the engineered multilayer vascular tube in a maturation chamber.

14. The method of claims 11-13, wherein one or more of the multicellular bodies of cells are elongate.

15. The method of claim 11, further comprising conditioning the cohered engineered multilayered vascular tube by pressurized intralumenal fluid perfusion.

16. The method of claim 15, wherein the intralumenal fluid perfusion is performed at pressures from 60 mm Hg to 200 mm Hg.

17. The method of claim 15, wherein the intraluminal fluid perfusion includes pulsatile forces.

18. The engineered multilayered vascular tube of claim 1 or 2, wherein the differentiated adult endothelial cells are human aortic endothelial cells.

19. An engineered vascular tube free from a pre-formed scaffold and formed through use of a bioprinter comprising differentiated adult smooth muscle cells, differentiated adult endothelial cells, and differentiated adult fibroblasts, wherein said cells are cohered to one another, wherein said engineered vascular tube has a ratio of endothelial cells to smooth muscle cells of 5:95 to 45:55, and wherein said tube has one, two, or all of the following features: (a) internal diameter of the engineered vascular tube is 6 mm or smaller; (b) the length of the engineered vascular tube is up to 30 cm; and (c) the thickness of the engineered vascular tube is substantially uniform along a region of the tube.

## Patentansprüche

1. Eine technische mehrschichtige Gefäßröhre, die unter Verwendung eines Bioprinters geformt ist, und mindestens eine Schicht aus differenzierten adulten Fibroblasten und mindestens eine Schicht aus differenzierten adulten glatten Muskelzellen umfasst, wobei entweder die mindestens eine Schicht der differenzierten adulten Fibroblasten oder die mindestens eine Schicht aus differenzierten adulten glatten Muskelzellen ferner differenzierte adulte Endothelzellen umfasst, wobei die technische mehrschichtige Gefäßröhre ein Verhältnis von Endothelzellen zu glatten Muskelzellen von 5:95 bis 45:55 aufweist, und wobei die technische mehrschichtige Gefäßröhre eine, zwei, drei oder alle der folgenden Eigenschaften hat: (a) Die technische mehrschichtige Gefäßröhre ist konform; (b) der Innendurchmesser der technischen mehrschichtigen Gefäßröhre ist 6 mm oder kleiner; (c) die Länge der technischen mehrschichtigen Gefäßröhre beträgt bis zu 30 cm; und (d) die Stärke der technischen mehrschichtigen Gefäßröhre ist im Wesentlichen entlang eines Bereichs der Röhre einheitlich; vorausgesetzt, die technische mehrschichtige Gefäßröhre ist frei von vorgeformten Zellträgern.

2. Die technische mehrschichtige Gefäßröhre nach Anspruch 1, umfassend eine Außenschicht aus differenzierten adulten Fibroblasten, und mindestens eine Innenschicht aus differenzierten adulten glatten Muskelzellen und differenzierten adulten Endothelzellen.

3. Die technische mehrschichtige Gefäßröhre nach Anspruch 1 oder 2, wobei die Berstdruckfestigkeit ausreicht, um einem physiologischen Blutdruck standzuhalten.

4. Die technische mehrschichtige Gefäßröhre nach einem der Ansprüche 1 bis 3, wobei das Verhältnis der Endothelzellen zu den glatten Muskelzellen 15:85 beträgt.

5. Die technische mehrschichtige Gefäßröhre nach einem der vorhergehenden Ansprüche, wobei der Innendurchmesser der technischen mehrschichtigen Gefäßröhre 0,5 mm oder kleiner ist.

6. Die technische mehrschichtige Gefäßröhre nach einem der vorhergehenden Ansprüche, wobei die Stärke der technischen mehrschichtigen Gefäßröhre im Wesentlichen über die Länge der Röhre einheitlich ist.

7. Die technische mehrschichtige Gefäßröhre nach einem der vorhergehenden Ansprüche, wobei die technische mehrschichtige Gefäßröhre im Wesentlichen frei von nichtdifferenzierten adulten Fibroblasten, nichtdifferenzierten adulten glatten Muskelzellen und/oder nichtdifferenzierten adulten Endothelzellen ist und/oder wobei die Röhre eine verzweigte Struktur hat.

8. Die technische mehrschichtige Gefäßröhre nach einem der vorhergehenden Ansprüche, wobei die Röhre nichtinnerviert ist, und/oder wobei die besagten differenzierten adulten Fibroblasten nichtvaskuläre Fibroblasten sind.

9. Die technische mehrschichtige Gefäßröhre nach Anspruch 3, für die Verwendung
a) bei Drogentests oder Tests von kardiovaskulären Geräten, optional wobei das kardiovaskuläre Gerät ein Stent ist; oder
b) bei der Reparatur von beschädigten Blutgefäßen; oder
c) bei der Behandlung eines Patienten, der einen koronaren oder peripheren Bypass oder arteriovenösen Zugang benötigt; oder
d) bei der Behandlung eines Patienten mit einer Nierenerkrankung im Endstadium, Diabetes, Arteriosklerose oder angeborenem Herzfehlern.

10. Die technische mehrschichtige Gefäßröhre nach Anspruch 9, wobei die Fibroblasten, die glatten Muskelzellen und/oder Endothelzellen der technischen mehrschichtigen Gefäßröhre autolog sind.

11. Ein Verfahren für die Bildung einer technischen mehrschichtigen Gefäßröhre, umfassend: das Positionieren mindestens eines Körpers einer Füllermatrix, mindestens eines mehrzelligen Körpers aus glatten Muskelzellen, wobei die Zellen miteinander verbunden sind, mindestens einen mehrzelligen Körper aus Endothelzellen, wobei die Zellen miteinander verbunden sind, und mindestens einen mehrzelligen Körper aus Fibroblastenzellen, wobei die Zellen miteinander verbunden sind, um eine gewünschte technische mehrschichtige Gefäßröhrenstruktur zu bilden, wobei das Verhältnis der Endothelzellen zu den glatten Muskelzellen in der technischen mehrschichtigen Gefäßöhre 5:95 bis 45:55 beträgt, und wobei ein oder mehrere Füllermatrix-Körper das Lumen der technischen mehrschichtigen Gefäßröhre bilden, und eine Vielzahl von Füllermatrix-Körpern die technische mehrschichtige Gefäßröhre umgeben; und das Erlauben, dass sich die mehrzelligen Körper der technischen mehrschichtigen Gefäßröhre miteinander verbinden; vorausgesetzt, dass zu keiner Zeit ein vorgeformter Zellträger verwendet wird, und vorausgesetzt, dass die technische mehrschichtige Gefäßröhre unter Verwendung eines Bioprinters gebildet wird.

12. Das Verfahren nach Anspruch 11, wobei der Durchmesser des Lumen der technischen mehrschichtigen Gefäßröhre 6 mm oder kleiner ist.

13. Das Verfahren nach Anspruch 11, ferner umfassend das Kultivieren der technischen mehrschichtigen Gefäßröhre in einer Reifungskammer.

14. Das Verfahren nach Anspruch 11-13, wobei einer oder mehrere der mehrzelligen Zellkörper länglich sind.

15. Das Verfahren nach Anspruch 11, ferner umfassend das Konditionieren der zusammengefügten technischen mehrschichtigen Gefäßröhre durch unter Druck stehende intraluminale Flüssigkeitsperfusion..

16. Das Verfahren nach Anspruch 15, wobei die intraluminale Flüssigkeitsperfusion bei Druck von 60 mm Hg bis 200 mm Hg durchgeführt wird.

17. Das Verfahren nach Anspruch 15, wobei die intraluminale Flüssigkeitsperfusion pulsierende Kräfte beinhaltet.

18. Die technische mehrschichtige Gefäßröhre nach Anspruch 1 oder 2, wobei die differenzierten adulten Endothelzellen menschliche aortische Endothelzellen sind.

19. Eine technische Gefäßröhre frei von vorgeformten Zellträgern und gebildet unter Verwendung eines Bioprinters, der differenzierte adulte glatte Muskelzellen, differenzierte adulte Endothelzellen und differenzierte adulte Fibroblasten umfasst, wobei die besagten Zellen miteinander verbunden sind, wobei die technische Gefäßröhre ein Verhältnis der Endothelzellen zu den glatten Muskelzellen von 5:95 bis 45:55 hat, und wobei die besagte Röhre eine, zwei oder alle der folgenden Eigenschaften hat: (a) Innendurchmesser der technischen Gefäßröhre ist 6 mm oder kleiner; (b) die Länge der technischen Gefäßröhre ist bis zu 30 cm; und (c) die Stärke der technischen Gefäßröhre ist im Wesentlichen entlang eines Bereichs der Röhre einheitlich.

## Revendications

1. Un tube vasculaire multicouche modifié formé par l'intermédiaire de l'utilisation d'une bio-imprimante comprenant au moins une couche de fibroblastes adultes différenciés et au moins une couche de cellules musculaires lisses adultes différenciées, dans lequel, soit ladite au moins une couche de fibroblastes adultes différenciés ou ladite au moins une couche de cellules musculaires lisses différenciées comprend en outre des cellules endothéliales adultes différenciées, dans laquelle ledit tube vasculaire multicouche modifié présente un rapport entre les cellules endothéliales et les cellules musculaires lisses de 5:95 à 45:55, et dans lequel ledit tube vasculaire multicouche modifié présente un, deux, trois, ou toutes les fonctions suivantes : (a) le tube vasculaire multicouche modifié est élastique ; (b) le diamètre intérieur du tube vasculaire multicouche modifié est inférieur ou égal à 6 mm ; (c) la longueur du tube vasculaire multicouche modifié peut atteindre environ 30 cm ; et (d) l'épaisseur du tube vasculaire multicouche modifié est pratiquement uniforme tout au long d'un segment du tube ; à condition que ledit tube vasculaire multicouche modifié ne comporte aucun tuteur préformé.

2. Tube vasculaire multicouche modifié selon la revendication 1 comprenant une couche externe de fibroblastes adultes différenciés et au moins une couche interne de cellules musculaires lisses adultes différenciées et de cellules endothéliales adultes différenciées.

3. Tube vasculaire multicouche modifié selon la revendication 1 ou 2, dans lequel la résistance à l'éclatement est suffisante pour résister à la pression sanguine physiologique.

4. Tube vasculaire multicouche modifié selon l'une quelconque des revendications 1 à 3, dans lequel le rapport entre les cellules endothéliales et les cellules musculaires lisses est de 15:85.

5. Tube vasculaire multicouche modifié selon l'une quelconque des revendications précédentes, dans lequel le diamètre interne du tube vasculaire multicouche modifié est inférieur ou égal à 0,5 mm.

6. Tube vasculaire multicouche modifié selon l'une quelconque des revendications précédentes, dans lequel l'épaisseur du tube vasculaire multicouche modifié est pratiquement uniforme sur la longueur du tube.

7. Tube vasculaire multicouche modifié selon l'une quelconque des revendications précédentes, le tube vasculaire multicouche modifié ne comportant pratiquement exempt de fibroblaste adulte non différencié, de cellules musculaires lisses adultes non différenciées et/ou de cellules endothéliales adultes non différenciées et/ou dans lequel le tube présente une structure ramifiée.

8. Tube vasculaire multicouche modifié selon l'une quelconque des revendications précédentes, dans lequel le tube est non innervé et/ou où lesdits fibroblastes adultes différenciés sont des fibroblastes non vasculaires.

9. Tube vasculaire multicouche modifié selon la revendication 3, destiné à l'utilisation dans
a) des tests de dépistage de drogue ou des tests de dispositifs cardiovasculaires, dans lequel éventuellement le dispositif cardiovasculaire est une endoprothèse ; ou
b) la réparation des vaisseaux sanguins endommagés ; ou
c) le traitement d'un patient qui a besoin d'une dérivation coronaire ou périphérique ou d'un accès artérioveineux ; ou
d) le traitement d'un patient étant en phase terminale d'une maladie rénale, atteint du diabète, d'artériosclérose, ou d'anomalies congénitales cardiaques congénitales.

10. Tube vasculaire multicouche modifié selon la revendication 9, dans lequel les fibroblastes, les cellules musculaires lisses et/ou les cellules endothéliales du tube vasculaire multicouche modifié sont un autologue.

11. Procédé de formation d'un tube vasculaire multicouche modifié comprenant :
le positionnement d'au moins un corps de matrice de remplissage, d'au moins un corps multicellulaire de cellules musculaires lisses dans lequel les cellules sont en adhésion l'une à l'autre, d'au moins un corps multicellulaire des cellules endothéliales dans laquelle les cellules sont en adhésion l'une à l'autre et d'au moins un corps multicellulaire de fibroblastes où les cellules sont en adhésion l'une à l'autre pour former une structure de tube vasculaire multicouche modifié souhaitée, dans laquelle le rapport entre les cellules endothéliales et les cellules musculaires lisses dans le tube vasculaire multicouche modifié est de 5:95 à 45:55, dans laquelle un ou plusieurs corps de matrice de remplissage forment le lumen du tube vasculaire multicouche modifié et une pluralité de corps de matrice de remplissage entourent le tube vasculaire multicouche modifié ; et permettant aux corps multicellulaires du tube vasculaire multicouche modifié d'adhérer ; à condition qu'aucun tuteur préformé ne soit utilisé en tout temps, et à condition que le tube vasculaire multicouche modifié soit formé grâce à l'utilisation d'une bio-imprimante.

12. Procédé selon la revendication 11, dans lequel le diamètre du lumen du tube vasculaire multicouche modifié est inférieur ou égal à 6 mm.

13. Procédé selon la revendication 11, comprenant en outre la culture du tube vasculaire multicouche modifié dans une chambre de maturation.

14. Procédé selon les revendications 11 à 13, dans lequel un ou plusieurs des corps multicellulaires de cellules sont allongés.

15. Procédé selon la revendication 11, comprenant en outre le conditionnement du tube vasculaire multicouche modifié en adhésion par perfusion de fluide intraluminal pressurisé.

16. Procédé selon la revendication 15, dans lequel la perfusion de fluide intraluminal est effectuée à des pressions de 60 mm Hg à 200 mm Hg.

17. Procédé selon la revendication 15, dans lequel la perfusion de fluide intraluminal comprend des forces pulsatiles.

18. Tube vasculaire multicouche modifié selon la revendication 1 ou 2, dans lequel les cellules endothéliales adultes différenciées sont des cellules endothéliales de l'aorte humaine.

19. Tube vasculaire modifié exempt d'un tuteur préformé et formé grâce à l'utilisation d'une bio-imprimante comprenant des cellules musculaires lisses adultes différenciées, des cellules endothéliales adultes différenciées et des fibroblastes adultes différenciés, dans laquelle lesdites cellules sont en adhésion l'une à l'autre, dans laquelle ledit tube vasculaire multicouche modifié présente un rapport entre les cellules endothéliales et les cellules musculaires lisses de 5:95 à 45:55 et dans lequel ledit tube a une, deux, ou toutes les fonctions suivantes : (a) le diamètre interne du tube vasculaire multicouche modifié est inférieur ou égal à 6 mm ; (b) la longueur du tube vasculaire multicouche modifié peut atteindre environ 30 cm ; et (c) l'épaisseur du tube vasculaire multicouche modifié est pratiquement uniforme tout au long d'un segment du tube.
